(19) **Europäisches Patentamt / European Patent Office / Office européen des brevets**

(11) **EP 1 613 253 B1**

(12) **EUROPEAN PATENT SPECIFICATION**

(45) Date of publication and mention
of the grant of the patent:
**31.12.2014   Bulletin 2015/01**

(21) Application number: **04759430.4**

(22) Date of filing: **12.04.2004**

(51) Int Cl.:
**A61F 9/01** (2006.01)

(86) International application number:
**PCT/US2004/011188**

(87) International publication number:
**WO 2004/091458 (28.10.2004 Gazette 2004/44)**

(54) **METHOD AND SYSTEM RELATED TO TREATMENT PLANNING FOR VISION CORRECTION**

VERFAHREN UND SYSTEM IM ZUSAMMENHANG MIT DER BEHANDLUNGSPLANUNG ZUR SEHKORREKTUR

PROCEDE ET SYSTEME ASSOCIE A LA PLANIFICATION DE TRAITEMENT POUR LA CORRECTION DE LA VISION

(84) Designated Contracting States:
**DE ES FR GB IT**

(30) Priority:  **11.04.2003   US 462649 P**

(43) Date of publication of application:
**11.01.2006   Bulletin 2006/02**

(73) Proprietor: **Technolas Perfect Vision GmbH**
**80992 München (DE)**

(72) Inventors:
• **FELBERG, Craig, L.**
**Laguna Niguel, CA 92677 (US)**
• **HUGHES, Robin**
**Rochester, NY 14608 (US)**
• **VEITH, Rupert**
**Rochester, NY 14604 (US)**
• **JOHNSON, Michael, Keith**
**Ogden, UT 84404 (US)**
• **DAMBACHER, Florian**
**Rochester, NY 14604 (US)**

• **MATTHAUS, Arne**
**Rochester, NY 14604 (US)**
• **YOUSSEFI, Gerhard**
**84028 Landshut (DE)**
• **MORITZ, Friedrich**
**Mission Viejo, CA 92692 (US)**

(74) Representative: **Vossius & Partner**
**Siebertstrasse 4**
**81675 München (DE)**

(56) References cited:
**WO-A1-98/48746         US-A- 6 159 205**
**US-A1- 2002 075 451**

• **GATINEL DAMIEN ET AL: "Three-dimensional representation and qualitative comparisons of the amount of tissue ablation to treat mixed and compound astigmatism." JOURNAL OF CATARACT AND REFRACTIVE SURGERY. NOV 2002, vol. 28, no. 11, November 2002 (2002-11), pages 2026-2034, XP002293300 ISSN: 0886-3350**

**Description**

BACKGROUND OF THE INVENTION

Field of the Invention

The invention is generally directed to the field of laser vision correction and more particularly to a method and a system for aiding in the identification and selection of a treatment plan for correcting vision in a patient's eye.

Description of Related Art

Since the earliest days of photoablative laser correction of vision defects *via* procedures referred to as PRK and LASIK, these treatments and more recently, LASEK, for example, have developed in terms of accuracy and scope of application. In the early days, subjectively measured refraction was coupled with crude (by today's standards) anterior corneal profile measurements to determine a treatment ablation based upon a naïve shape subtraction model of the cornea delivered by a broad beam, fixed axis laser beam. Over the past fifteen years, more advanced lasers have been developed that employ small spots at a high repetition based according to complex shot sorting and sequencing calculations to ablate more accurately, more efficiently, and much more correctively than in the past. Advanced topography technology, wavefront aberration measurement and analysis, laser pachymetry, and other diagnostic techniques and instrumentation have driven the development of complex treatment algorithms that no longer merely correct a patient's manifest refraction to improve visual acuity, but rather to correct higher order aberrations, compensate for biodynamic responses of the eye to tissue destruction processes, compensate for thermal heating effects on the cornea due to the bombardment by laser pulses, and adjust for ablation beam efficiency due to oblique beam placement locations. Moreover, countless nomogram adjustments are applied to treatment algorithms to account for various myopic, hyperopic, environmental, biographic, and other parameters that effect vision correction. The aimed for end result of all of this is supervision.

With the rapid advances in technology and knowledge comes concomitant challenges for the surgeon to decide which of the many available laser platforms will execute the optimum treatment plan for a particular vision defect in a uniquely characterized eye. One might reasonably conclude that the kind of applied treatment depends upon the pre-operative findings of the patient's eye, which in itself leads to a myriad of choices. For example, an eye having significant higher order aberrations may be an appropriate candidate for a customized ablation procedure based upon wavefront data. The same eye, however, could also be a candidate for a topography-driven treatment if the main aberrations are caused in the anterior corneal surface.

Regardless of skill level, the surgeon has available only a few decision criteria to decide what kind of treatment should best be applied to the patient's eye. If, for example, three treatment options are available in which one may optimize optical zone but at the expense of tissue consumption, while another may improve visual acuity but result in poor contrast sensitivity under low light conditions, and the third provide yet different tradeoffs, it quickly becomes obvious that even out of the particular treatment options available to the surgeon, only a very few can realistically be considered.

Accordingly, the inventors have recognized the need for, and advantages of, a method and system that would aid the surgeon in classifying a particular patient's eyes and in selecting a treatment plan based upon a review of a multiplicity of viable treatment plans that can automatically and simultaneously be computed, optimized, and displayed to the surgeon for review and selection. Thus, there is a recognized need for a solution to the problems discussed hereinabove and related thereto which are advantageously addressed by the instant invention set forth in the following description and the appended claims.

US 2002/0075451 A1 discloses a system and method for correcting corneal irregularities through the shaping of an eye's cornea to provide a desired corrective corneal curvature. The system may include an interface system, which can be a stand alone item, providing a tool for use by a surgeon or the like which allows a surgeon to input his expertise in the development of a clinical ablation profile that is well suited for the eye characteristics, review and also simulate a wide variety of potential surgical alternatives for a wide variety of corneal defects including irregular eye shapes and corneal surface irregularities.

SUMMARY OF THE INVENTION

The invention is directed to a system and methods for automatically determining a multiple number of viable treatment plans for correcting a patient's vision *via* photoablative refractive surgery. Embodiments of the invention rely on selected various diagnostic input about the patient's eye to classify the eye as being particularly suitable for treatment by several different treatment algorithms selected from a larger group of available treatment algorithms. The invention is further directed to the simultaneous presentation of various treatment plans based upon selected input data and available treatment algorithms that can be reviewed, modified, and ultimately selected by the surgeon for application to the patient's eye.

An algorithm to aid the surgeon in the selection of a treatment plan for vision correction in a patient's eye may include the steps of acquiring selected diagnostic input data types about the patient's eye, parameterizing the input data to automatically classify the patient's eye into one of several pre-determined classification sets, automatically determining two or more viable treatment algorithms suitable for correcting the patient's vision defects based upon the classification of the patient's eye, and presenting two or more corresponding treatment plans to the surgeon for prospective selection of one of the treatment plans.

The initial treatment algorithm calculations are based upon outcome determinative default parameters. The surgeon may selectively modify any or all of the default parameters and review re-calculated treatment plans, one of which may be selected to correct the patient's vision.

The diagnostic input data includes wavefront data, topography data, pachymetry data, refraction data, or other selected diagnostic information that is utilized either alone or in mutual combination by a variety of available treatment algorithms to treat the defects of the particularly classified eyes. The classification sets may include virgin eyes versus previously treated eyes, regular eyes versus irregular eyes, and myopic and/or hyperopic eyes with or without mixed astigmatism.

An embodiment of the invention is directed to a method as defined by claim 23 for aiding the selection of a treatment plan for correcting vision in a patient's eye. The method includes the steps of obtaining selected input diagnostic information about the patient's eye, analyzing the input diagnostic information to automatically determine two or more potentially usable treatment algorithms that are selected from an equal or larger number of available treatment algorithms, processing the potentially usable treatment algorithms using pre-set outcome determinative default parameters, presenting a number of viable treatment plans to the surgeon for review corresponding to the potentially usable treatment algorithms, selectively modifying one or more of the default parameters and other defined treatment parameters, re-processing the two or more potentially usable treatment algorithms using the modified parameters, and re-presenting to the surgeon for further review the corresponding treatment plans for correcting the patient's vision based upon the diagnostic input information.

In an aspect, the method further includes selecting one of the treatment plans for application to the patien's eye.

In another aspect, information in the calculated treatment plans can be optimized and sorted to allow the surgeon to compare the multiple viable treatment plans based upon the surgeon's preferred criteria.

In another aspect, the method includes the step of automatically recommending a preferred treatment plan or, alternatively, warning against contraindicated treatment plans.

In both of the foregoing process embodiments, the selection, processing, storage, and modification of various diagnostic, biographic, and therapeutic information, as well as the display and selection of viable treatment plans is accomplished through a multilevel graphical user interface (GUI).

A system embodiment according to the invention is defined by claim 1 and includes a component for receiving the diagnostic input data about the patient's vision, for analyzing the input data and determining the potentially usable treatment algorithms from a database comprising an equal or larger number of available treatment algorithms, and for processing the potentially usable treatment algorithms based upon the input data; and a component for displaying the multilevel graphical user interface which facilitates review, modification, and selection of viable treatment plans for correcting the patient's vision. The system is further operably associated with a storage medium for storing calculated and selected treatment plans which include executable instructions for a photoablative laser component of the system to deliver a selected treatment plan to the patient's eye. In an aspect, the receiving component is one of a variety of computing platforms well known in the art; the display component is a display monitor; the storage component is any of a variety of well known storage media including diskettes, CDs, DVDs, and the like; and the laser component is a 193nm excimer laser or other suitable laser for ablating corneal tissue. In an aspect, an eyetracker system and/or a microkeratome device and/or other diagnostic or therapeutic components are in operable communication with the laser system.

BRIEF DESCRIPTION OF THE DRAWINGS

Fig. 1 is a schematic flow diagram of an algorithm for classifying a patient's eye for aiding in the selection of a treatment plan for vision correction in a patient's eye according to an embodiment of the invention;

Fig. 2 is a schematic flow diagram of a method for aiding in the selection of a treatment plan for vision correction in a patient's eye according to another embodiment of the invention;

Fig. 3 is a schematic representation of a system used for planning a treatment for vision correction in a patient's eye according to an embodiment of the invention;

Fig. 4 is a schematic representation of a process step set forth in Fig. 2;

Fig. 5 is a schematic representation of another process step set forth in Fig. 2;

Fig. 6 is a view of a start-up display screen of a graphical user interface (GUI) according to an embodiment of the invention;

Fig. 7 is a view of another display screen of the GUI according to an embodiment of the invention;

Fig. 8 is a view of another display screen of the GUI according to an embodiment of the invention;

Fig. 9 is a view of another display screen of the GUI according to an embodiment of the invention;

Fig. 10 is a view of another display screen of the GUI according to an embodiment of the invention;

Fig. 11 is a view of another display screen of the GUI according to an embodiment of the invention;

Figs. 12a,b are views of a preferences display screen of the GUI according to an exemplary embodiment of the invention;

Fig. 13 is a view of a patient selection display screen of the GUI according to an exemplary embodiment of the invention;

Fig. 14 is a view of a patient information display screen of the GUI according to an exemplary embodiment of the invention;

Fig. 15 is a view of a treatment overview display screen of the GUI according to an exemplary embodiment of the invention;

Fig. 16 is a view of an illustrative treatment option display screen of the GUI according to an exemplary embodiment of the invention;

Fig. 17 is a view of another illustrative treatment option display screen of the GUI according to an exemplary embodiment of the invention;

Figs. 18a,b,c are views of a data check display screen of the GUI according to an exemplary embodiment of the invention; and

Fig. 19 is table of parameter value ranges according to an exemplary embodiment of the invention.

## DETAILED DESCRIPTION OF A PREFERRED EMBODIMENT OF THE INVENTION

To assist the reader in a clear understanding of the invention, certain terminology used throughout the description of the invention will be understood to have the following meanings: In the field of refractive laser vision correction, a treatment algorithm is understood as the process calculation for determining certain parameters of a particular type of treatment. For example, for a laser ablation treatment to correct a myopia refractive error, a desired or target corneal profile will be determined as well as the number, sequence, and placement of laser shots on the pre-operative corneal surface to obtain the target profile. The laser shot placement, sequence, and number calculations are parameters used to calculate a shot file, referred to herein as a ".TLS file," which stands for Technolas Laser Session. A laser used to apply the ablative photorefractive treatment uses the shot file as its executable instruction to carry out the particular treatment. A treatment plan as that term is used herein represents the process planning and result of processing a particular treatment algorithm based upon defined parameters and determining a prospective outcome represented by a variety of information that may include algorithm parameters as well as simulated post-operative maps, simulated ablation profiles, pachymetry data, optical zone dimensions, refraction values, aberration information, topography information, vision metric indicators, and other relevant information, including the shot file. Further as used herein, with respect to eye classification, a virgin eye means an eye that has not had prior corneal refractive surgery; a regular eye refers to an eye having associated vision defects that are correctable with standard ophthalmics such as spectacles, contact lenses, and the like, or by non-customized photoablative surgery limited to sphere and cylinder correction, whereas an irregular eye will be, for example, a keratoconic eye or an eye that cannot typically be characterized by wavefront measurements, but rather requires topographic or other gross analyses. Myopic, hyperopic, and astigmatic eyes have their typical meanings as well understood in the art.

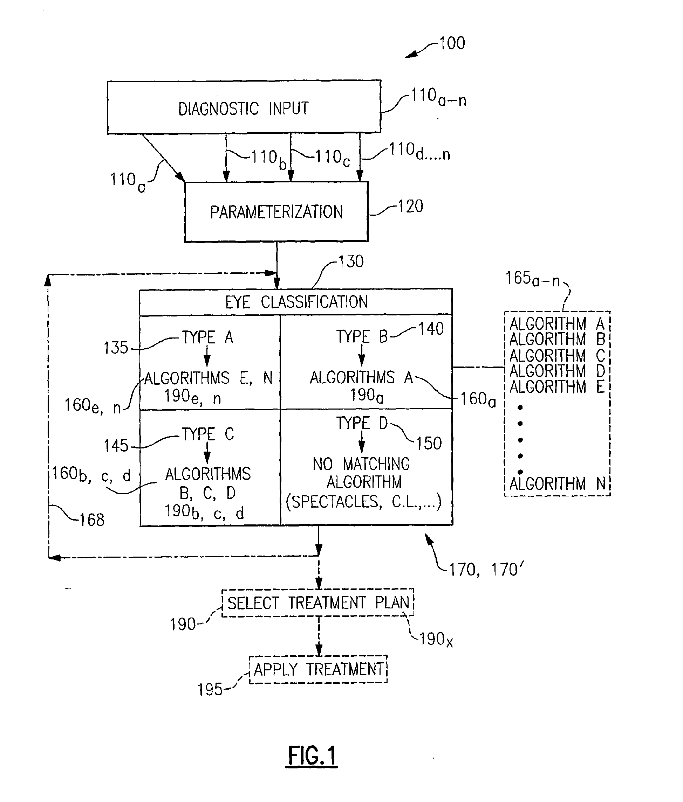

Fig. 1 illustrates an algorithm 100 to aid in the selection of a treatment plan 190 for vision correction in a patient's eye. At step 110 a variety of types of diagnostic input data $110_a$, $110_b$, $110_c$, $110_{d...}$ $110_n$ that has been acquired is provided as input to the processing software. These diagnostic data types include, for example, wavefront data, topography data, pachymetry data, refraction data, and other data types that a person skilled in the art could utilize to characterize a patient's eye and its vision defects. All, or only some, or various combinations of the data may be acquired and/or used as will be determined by the various treatment algorithms. At step 120 the diagnostic input data $110_n$ is parameterized so that the patient's eye can be classified into one of a pre-determined number of classification sets designated, for illustration purposes only, as Type A 135, Type B 140, Type C 145, and Type D 150. In an aspect according to the invention, the classification types are determined to be virgin eyes versus previously treated eyes, regular eyes versus irregular eyes, myopic eyes with or without astigmatism, particularly mixed astigmatism, and hyperopic eyes with or without simple and/or mixed astigmatism, as these terms have been defined hereinabove. It will be understood that the particular listed classification types correspond to types of eyes and/or vision defects associated with particular treatment algorithms that typically are proprietary to a supplier. For example, a treatment algorithm known to those skilled in the art as a Planoscan® treatment (Bausch & Lomb Incorporated, Rochester, New York) might be used to treat a regular, myopic or hyperopic eye using a Technolas 217® laser system (Bausch & Lomb Incorporated, Rochester, New York). Or, for example, a topographically driven treatment algorithm such as referred to in U.S. Patent No. 5,891,132 may suitably be used to treat an eye determined to be irregular by topographic diagnostic input data. In another example, an eye classified as having measured higher order aberrations and a regular topography might be a preferred candidate for a custom Zyoptix® treatment algorithm (Bausch & Lomb Incorporated, Rochester, New York). According to an exemplary embodiment of the invention, and as known in the art, it can be determined whether an eye has previously had photoablative laser surgery by looking at topographic and pachymetric diagnostic information; for example, by analyzing anterior surface topography and delta-pachymetry measurements, the presence and location of a transition zone in the previously ablated cornea can be determined. The irregularity of an eye for purposes of classification can be determined, for example, by examining the symmetry (dipole) of the corneal topography or decentration. Wavefront measurement and analysis, manifest refraction, and other techniques known to those skilled in the art can be used to determine the sign and magnitude of defocus and cylinder errors and higher order aberration information. Using these types of information to classify a patient's eye helps to identify which treatment algorithm(s) might be appropriate for developing a treatment plan to correct the patient's vision defect. Illustratively, a regular topography and a low wavefront measurement may indicate that a standard ablation is the suitable treatment. A regular topography and a high wavefront measurement may indicate that a customized or semi-customized wavefront-based treatment is most appropriate. An irregular topography plus a high wavefront measurement (i.e., highly aberrated cornea) may indicate that a topographically-based treatment or a hybrid-driven treatment should be considered. In this aspect, higher-order corneal aberrations, rather than the higher-order aberrations of the entire ocular system, are addressed. Irregular topography with or without a high wavefront measurement may contraindicate refractive surgery, suggesting instead spectacle or other ophthalmic lens correction, or ophthalmic surgical correction such as a corneal transplant, for example. Having thus described particular diagnostic types, eye classification types, treatment algorithm types, and so on, it is to be understood that the invention is in no way limited by the foregoing examples.

At step 130 all of the viable treatment algorithms $160_{a-n}$ for the particular eye classifications are determined from a pre-programmed database of all available treatment algorithms $165_{a-n}$. For example, the patient's eye may be classified by refraction as a Type A having typical myopia with astigmatism, that may suitably be treated by treatment algorithm $160_e$ or treatment algorithm $160_n$. At step 170, a multiple number of treatment plans $190_{a-n}$ are presented in the form of a display to be discussed in greater detail below, that respectively correspond to the appropriately identified treatment algorithms $160_{a-n}$ using pre-programmed default parameters which are outcome determinative of the algorithms.

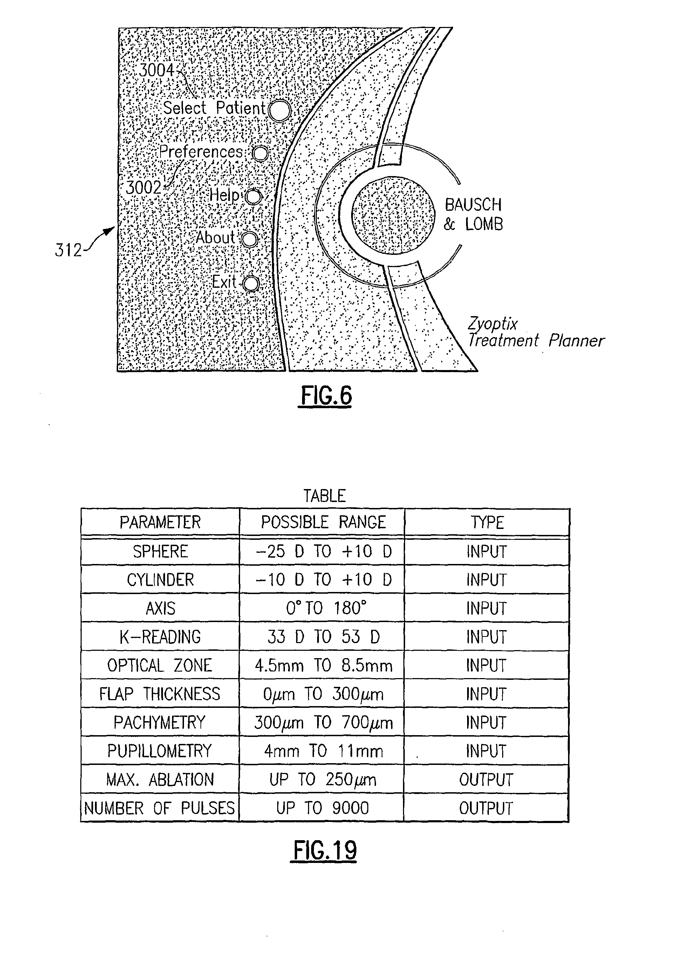

The multiplicity of treatment plans $190_{a-n}$ based upon the default parameters and the diagnostic input data will be presented to the surgeon *via* a display screen in the form of a multi-level graphical user interface (GUI) 312-316 illustrated by examples in Figs. 6-11. According to the invention, the surgeon then has the option to a) review the various treatment plans ($190_{a-n}$) that have been presented, b) save these to a storage medium (282), c) select one of the treatment plans ($190_x$) for prospective application, or d) modify (step 168) selected parameters and be presented with re-calculated treatment plans ($190'_{a-n}$), as will be described in greater detail below in respect to a related embodiment according to the invention.

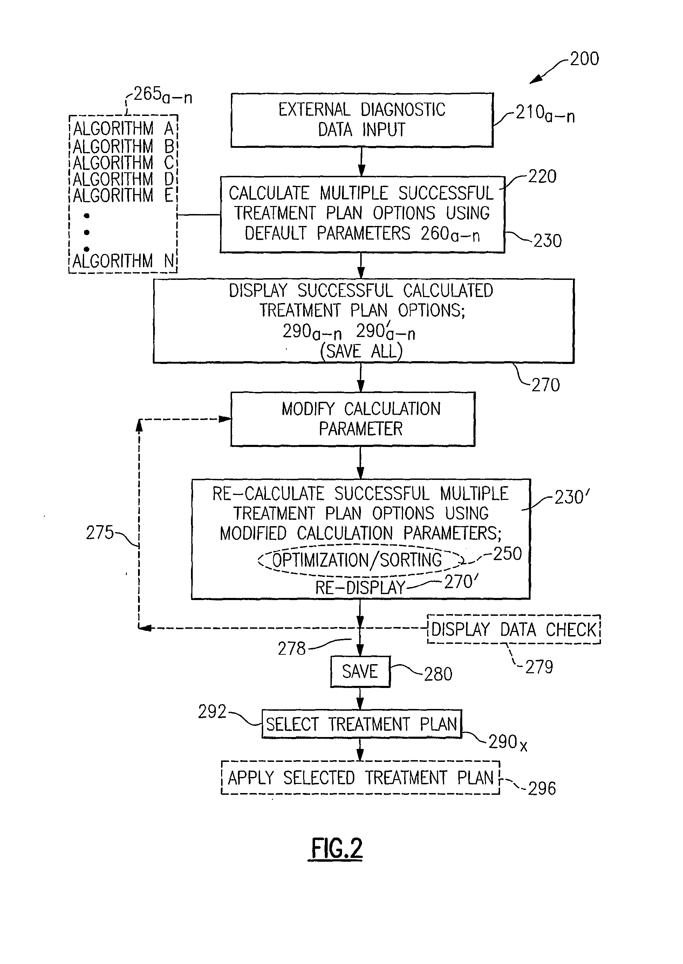

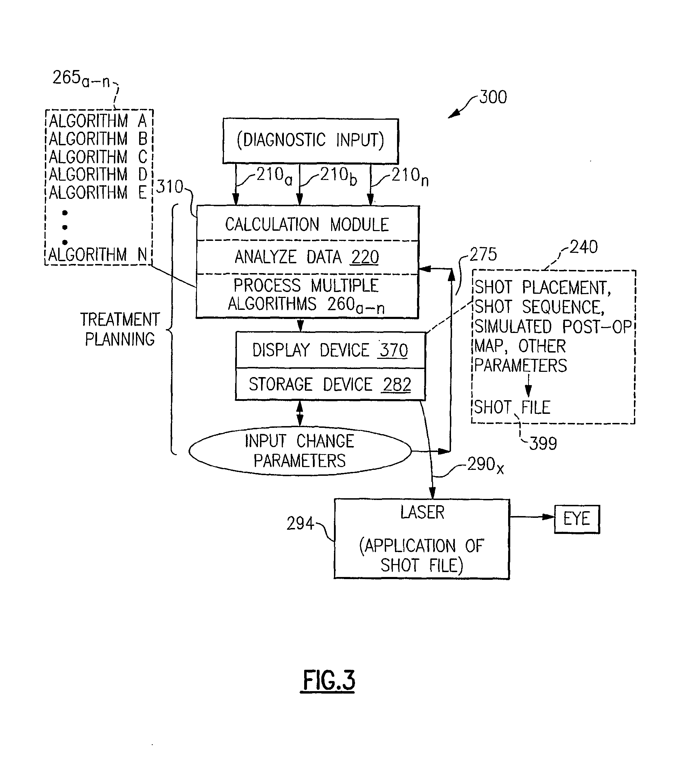

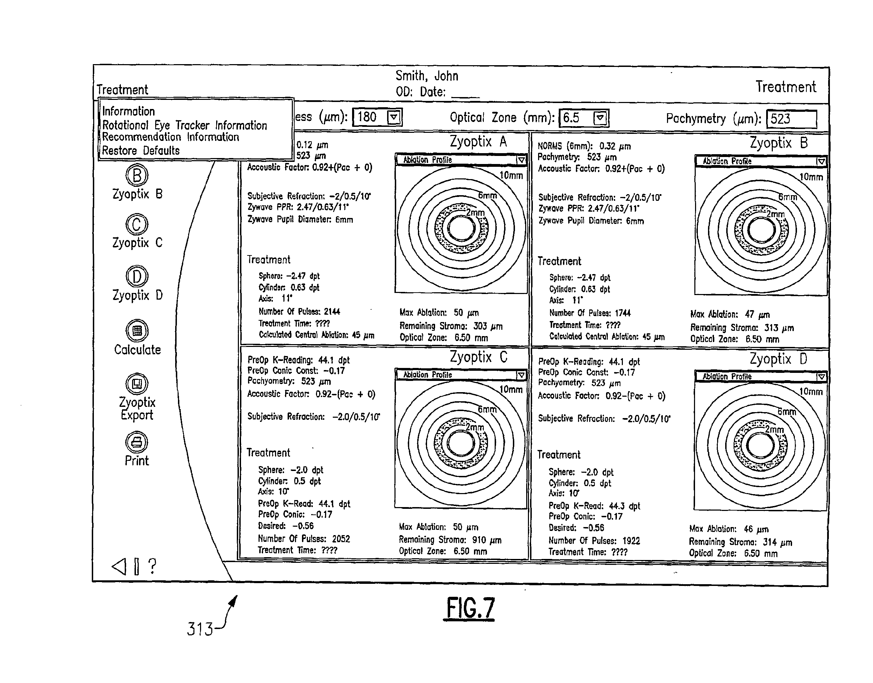

Another embodiment according to the invention is now described with reference to Figs. 2-11. Fig. 2 diagrammatically sets forth a method 200 for aiding the selection of a treatment plan $290_x$ for correcting vision in a patient's eye. At step 210, selected pre-operative diagnostic data $210_{a-n}$ that has been obtained is provided to a calculation module 310 (Fig. 3) where, at step 220 it is analyzed to determine a multiple number of potentially usable treatment algorithms $260_{a-n}$ selected from a database containing an equal or larger number of available treatment algorithms $265_{a-n}$ which are initially programmed with certain outcome determinative default parameters to allow first-run calculations. As described above, the analysis of particular diagnostic data $210_{a-n}$ will inform the selection of particular treatment algorithms $260_{a-n}$, leading to available treatment plans $290_{a-n}$. According to the invention, this association is done automatically for all potentially usable treatment algorithms selected from a database of all available algorithms $265_{a-n}$. At step 230 the calculation module processes the potentially usable treatment algorithms $260_{a-n}$ utilizing various outcome determinative default parameters; for example, LASIK flap thickness, residual stromal tissue depth, optical zone size, and/or others to determine the corresponding plurality of treatment plans $290_{a-n}$. These various treatment plans are displayed to the surgeon at step 270 on a display device 370 (Fig. 3) by means of the multi-level graphical user interface (GUI) 312 (Fig. 6) and illustrated in various display formats 313-316 in Figs. 7-11.

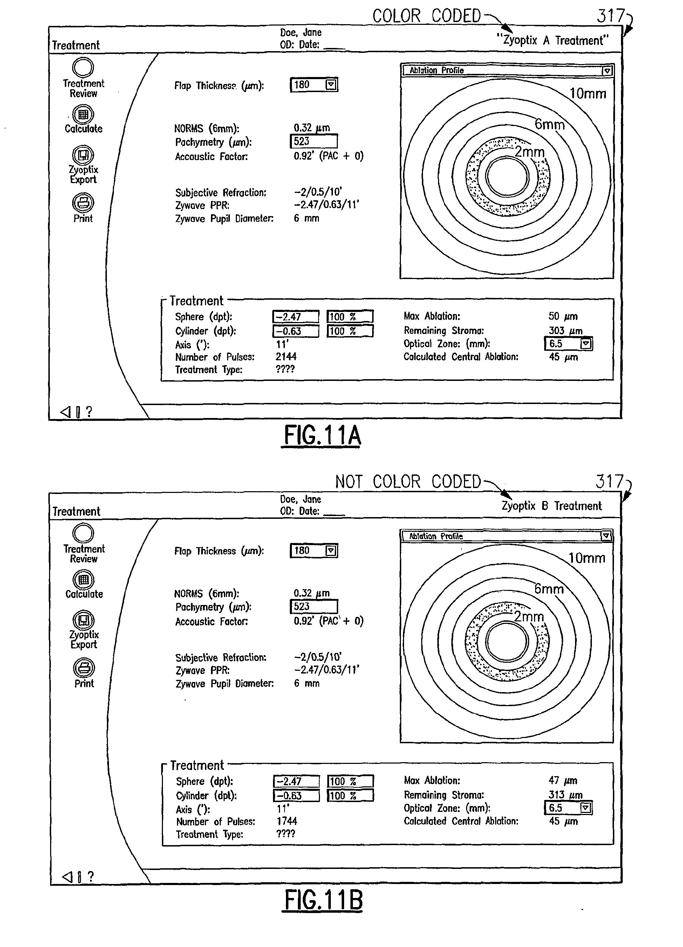

In Fig. 7, for example, four treatment plans $290_{a-d}$ representing a Zyoptix® A treatment algorithm, a Zyoptix B treatment algorithm, a Zyoptix C treatment algorithm, and a Zyoptix D treatment algorithm are illustrated on a GUI treatment review display 313. Each treatment plan $290_n$ contains a variety of information about the treatment including simulated ablation profile maps, various diagnostic measurements such as optical zone diameter and pachymetry, number of laser ablation shots and ablation depth for each treatment algorithm, and other information as can be seen in Fig. 7. In the exemplary embodiment of Fig. 7, for all of the treatments information is provided about pachymetry, subjective refraction, treatment refraction, number of laser pulses, treatment time, maximum ablation ($\mu$m), remaining stroma ($\mu$m), and optical zone diameter (mm). For the Zyoptix A and B treatment plans, information is presented regarding higher order aberrations over a 6mm pupil diameter, objective manifest refraction (sphere, cylinder, axis), pupil diameter (mm) for the calculated objective manifest refraction, and central ablation depth ($\mu$m). For the Zyoptix C and D treatment plans, the presented information includes pre-operative K-readings in diopters (D), pre-operative conic constants (Q), treatment pre-operative K-readings (D), and target post-operative conic constants (Q'). As will be discussed further below, the exemplary treatment review screen 313 in Figure 7 may color-code treatments that are recommended or contraindicated, as well as provide textual information as to why a particular treatment plan should or should not be used. The screen provides an editable field for "pachymetry," "flap thickness," and "optical zone" to allow adjustment of these values for all of the displayed treatments. Default values may also be restored for the above editable fields. Selectable information is provided relating to the software version of the system, and diagnostic wavefront and topography files with associated comments. A selectable item for rotational eye tracking information relating to pupil shift and pupil rotation is further provided. Recalculation of the available treatment plans based upon user input changes are calculated from this display screen. Treatment plans and associated graphical maps can be maximized as illustrated by the display screens in Fig. 11. Hint boxes can be displayed for each of the treatment plans based on cursor placement; for example, under Zyoptix A: "wavefront-based treatments with enhanced asphericity for myopia" (Zyoptix personalized aspheric treatment mode); Zyoptix B: "wavefront-based treatment" (Zyoptix personalized treatment mode); Zyoptix C: "aspheric treatment with reduced ablation depth" (Zyoptix aspheric tissue saving treatment mode); and Zyoptix D: "treatment with reduced ablation depth" (Zyoptix tissue saving treatment mode). The exemplary treatment review screen GUI will facilitate execution of storage and selection commands as well as display warning messages based upon modified default parameters. This screen further allows the export of the selected and calculated treatment to an executable .TLS file for laser application. Commands for optimization of the optical zone and other optimization/sorting functions are also executed from this screen.

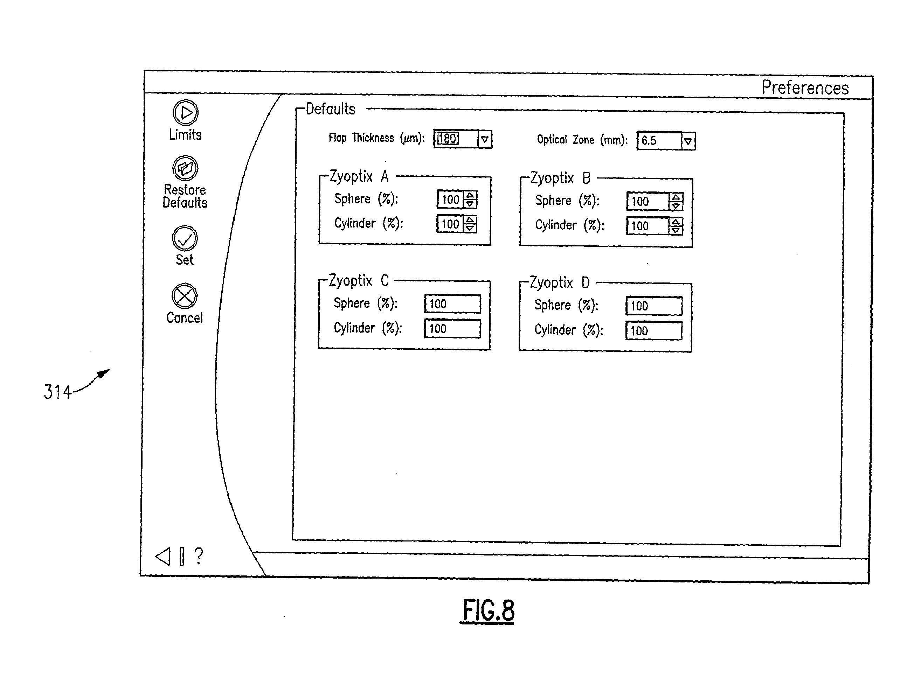

Due to the fact that each of the calculated treatment plans $290_{a-d}$ are based on default parameters that represent required initial input for the treatment algorithms, the method according to the invention provides for user selective modification of one or more of the default parameters and/or other defined treatment parameters at step 275 as illustrated by GUI screen display 314 in Fig. 8, accessed by a pull-down menu. The user can then request re-processing of the treatment algorithms shown at step 230' in Fig. 2 whereupon re-calculated treatment plans 290' are re-displayed at step 270'. The re-calculated treatment plans $290'_{a-d}$ appear as illustrated again by 313 in Fig. 7. This type of iteration can occur as many times as the surgeon feels is necessary, but a single iteration will likely be sufficient in most cases.

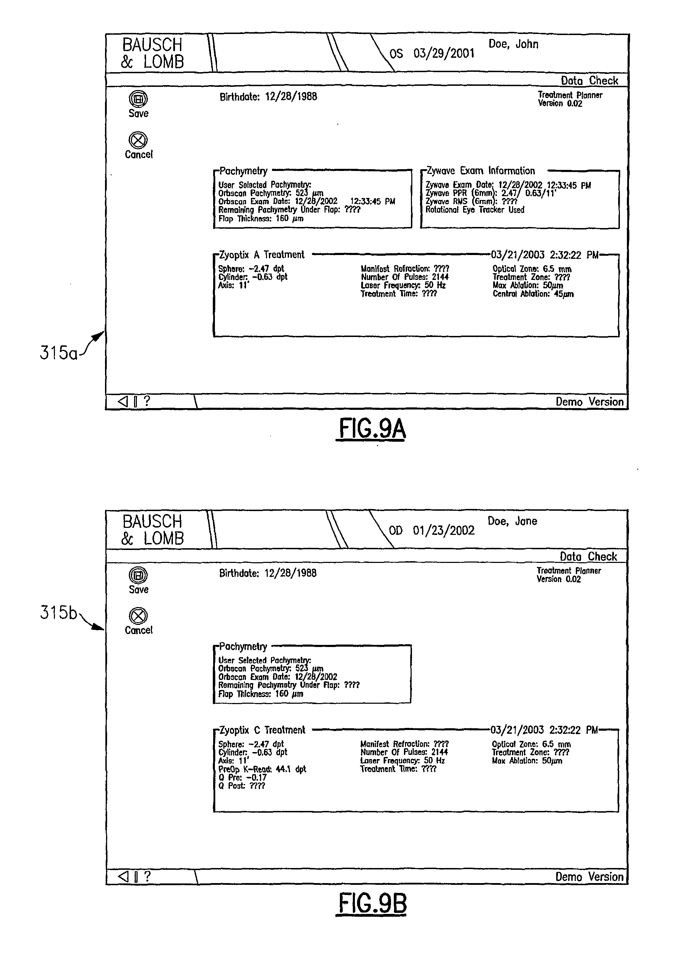

In an aspect according to this embodiment, the user can save the calculated treatment plans at step 280 in a storage medium 282 (Fig. 3) that is readable or has means making it readable by a therapeutic component 294 such as an excimer laser used to apply the ultimately selected treatment plan $290_x$. As described above, each stored treatment plan $290_{a-n}$ will ultimately contain a shot file 399 which is the executable instruction carried out by the therapeutic laser component to apply the desired photoablation treatment to the eye. Prior to saving at step 280, the GUI displays a data check screen at step 279 as illustrated by displays 315a,b in Figs. 9A and 9B for two different patients. Each data check screen 315 contains information based upon the calculated and selected treatment plans. In a first exemplary embodiment illustrated in Fig. 9B, the data check screen 315b contains the following information: patient name, patient date of birth, patient eye, treatment algorithm type, selected treatment sphere, selected treatment cylinder, selected treatment axis, optical zone size, treatment zone, number of laser pulses, treatment time, maximum ablation depth, remaining pachymetry under the flap for the specified flap thickness (LASIK), currently running software version, calculation date, diagnostic exam dates, instrument-specific pachymetry informations, manifest refraction, and laser shot frequency. In another exemplary embodiment illustrated in Fig. 9A, the information in the data check screen 315a includes objective manifest refraction, central ablation depth, wavefront RMS values at 6mm pupil diameter, rotational eyetracker data (if used), and a text message such as "recommended centration of treatment is the pupil center". In a third exemplary embodiment for particular treatment plan types, the data check information consists of corneal curvature information (K-readings) and pre- and post-operative conic shape profiles (Q-factors).

Once the suitable treatment plans are saved at step 280, a treatment plan $290_x$ is selected at step 292 for potential application at step 296.

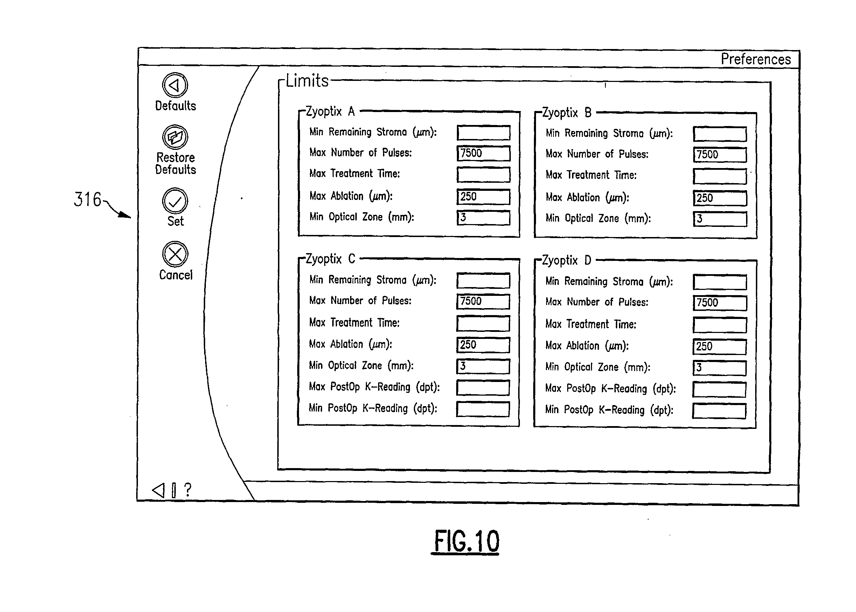

Another aspect of the GUI display structure and function is a preference screen 316 illustrated in Fig. 10 that is also accessed *via* a pull-down menu. In an exemplary embodiment, the preference screen allows: (a) default nomogram values to be set for all different treatment options; (b) the setting of a default assumed flap thickness (LASIK) for the different treatment options; (c) setting of a default optical zone diameter for different treatment options; (d) the setting of limit values for maximum ablation, treatment time, number of laser pulses, minimal optical zone size, minimum post-operative K-reading, maximum post-operative K-reading, and minimum remaining stromal thickness under the flap (LASIK); and (e) a fixed amount of over or under correction can be set as a nomogram value for a particular treatment (e.g., add 0.5D to the sphere for all Type A treatment plans).

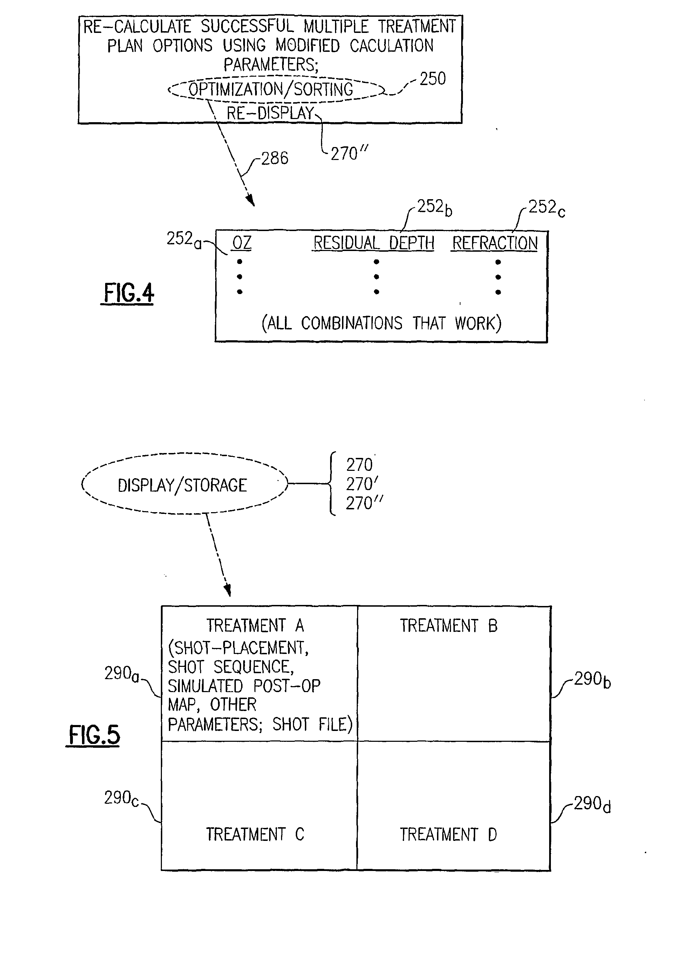

In another aspect, with reference to Fig. 4, the surgeon may wish to re-evaluate the available treatment plans based upon certain optimized parameters. For example, for the Zyoptix A-D treatment plans illustrated in Fig. 7, it may be desirable to view all of the treatment plan information based upon the optimization of optical zone (OZ) diameter. In the treatment review screens 313 illustrated in Fig. 7, for example, an input to optimize the OZ is provided. When executed, the optical zone is increased for all available treatment plans until the minimum residual stromal depth under the flap (LASIK) is equal to a user-defined limit, to the extent allowable based upon diagnostic wavefront input data for example. At step 250 in Fig. 2, optimization and sorting illustrated by OZ $252_a$, residual stromal depth $252_b$, and refraction $252_c$ can be calculated and treatment plans re-displayed at step 270" as shown in Fig. 4.

In another aspect, the number of potentially useable treatment algorithms $260_{a-n}$ is at least two selected from a larger group of available treatment algorithms $265_{a-n}$ (Fig. 2) preferably directed to myopia treatment only, hyperopia treatment only, myopia with astigmatism, hyperopia with astigmatism, other standard lower order aberration correction, higher order aberration correction, re-treatment correction, spherical collective treatment with reduced tissue ablation, aspheric corrective treatment with reduced ablation volume, LASIK treatments, LASEK treatments, PRK treatments, nomogram adjusted treatments, and other customized treatments. The invention, however, is not so limited.

In another aspect according to an embodiment of the invention, the system may be programmed to automatically recommend a preferred treatment plan to the user. This option could be implemented by color-coding the preferred treatment plan out of the available treatment plans as illustrated by 317 in Fig. 11.

Based upon the nature of the treatment plan, certain other default data, preference data, warning information, and other outcome determinative parameters may be made accessible through the GUI menus. For example, the calculation of certain treatment plans for the correction of aspherical aberrations may require the input of rotational eyetracker information. LASIK-based treatments may require particular microkeratometric information.

A system embodiment 300 according to the invention is schematically illustrated in Fig. 3. Diagnostic input $210_n$ can be obtained by various topography devices, wavefront sensors, pachymetry devices, phoropters, customized diagnostic instrumentation, and other ophthalmic devices and techniques that are not in and of themselves parts of the invention *per se.* The means 310 for receiving the diagnostic input data, for analyzing the input data and determining the potentially usable treatment algorithms, and for processing the potentially usable treatment algorithms based upon the input data and other algorithm outcome determinative parameters can be a software-driven calculation module such as a P.C. or other well-known form of a computing platform. The display means 370 is typically a screen or monitor well known in the art, which is used to display the graphical user interface 312 (and associated display screens and functions) as described hereinabove. Once it is determined what functions and attributes are to be provided by the GUI according to the embodiments of the invention, it is well within the skill in the art of computer programming to create the appropriate GUI displays and calculation processes. A device-readable storage medium 282 will typically be a computer diskette, floppy disk, CD, DVD, computer hard drive, or electromagnetic carrier wave which can store the data in appropriate file form and which is readable by a computer or control component of the therapeutic laser component 294 of the system to execute the shot (.TLS) file 399 for applying the selected treatment plan $290_x$. The photoablative laser component 294 will typically be an excimer laser emitting light having a wavelength of 193nm or other suitable gas medium or solid state laser device adapted for corneal tissue photoablation.

Another exemplary embodiment according to the invention is illustrated by a treatment planning software calculation program (referred to hereinafter as "Treatment Planner") that generates an individual treatment file for a patient on the basis of the available measured data from diagnostic wavefront sensing measurements and diagnostic corneal topography and pachymetry measurements. In an exemplary aspect, the diagnostic information is accessed by a Zyoptix® Diagnostic Workstation (Bausch & Lomb Incorporated, Rochester, New York). This workstation is a combination Zywave II Aberrometer and Orbscan IIz Anterior Segment Analyzer. Zywave (aberrometer) data is stored in what are referred to as ".ATE" (i.e., Aberrometer Technolas Export) files, and Orbscan (corneal) data is stored in ".OTE" (i.e., Orbscan Technolas Export) files. The subsequently calculated treatment data is stored in the ".TLS" file, described above, which forms the basis for the refractive laser ablation treatment. According to an exemplary aspect, the treatment is known in the art as a Zyoptix vision correction treatment, which is carried out with a Technolas 217z excimer laser system operating at 100Hz.

According to the exemplary embodiment, the Treatment Planner calculation software is utilized by selection from a Windows® computer screen icon, which directs the user to the main screen display 312 as shown in Fig. 6. The user can click on the icon 3002 labeled "Preferences" to preset settings such as, for example, the file path for the wavefront diagnostic (.ATE) files, the corneal (.OTE) diagnostic files, and the treatment data (.TLS) files; a language preference; clinic information such as clinic name, laser serial number, surgeon name(s) and technician name(s). The Preferences screen also allows certain default settings to be preset; for example, LASIK flap thickness; K-reading; Q-value (e.g. Q, Q'), which represent a pre-operative aspheric corneal shape factor and a post-operative aspheric corneal shape factor, respectively; OZ (optical zone); OZ calculated from pupil size; and nomograms representing a percent of baseline refraction values. (K-reading is the central corneal curvature calculated on the basis of corneal elevation measurements and is expresses in diopter units as (n-1)/R, where R is the radius of the eye). The interested reader is referred to U.S. Application Serial No. 10/460801 entitled BICONIC ABLATION WITH CONTROLLED SPHERICAL ABERRATION, filed on June 12, 2003. Said application relates to an ablation treatment plan for an aspheric tissue saving mode that may be one of the treatment modes of the instant invention embodiment.

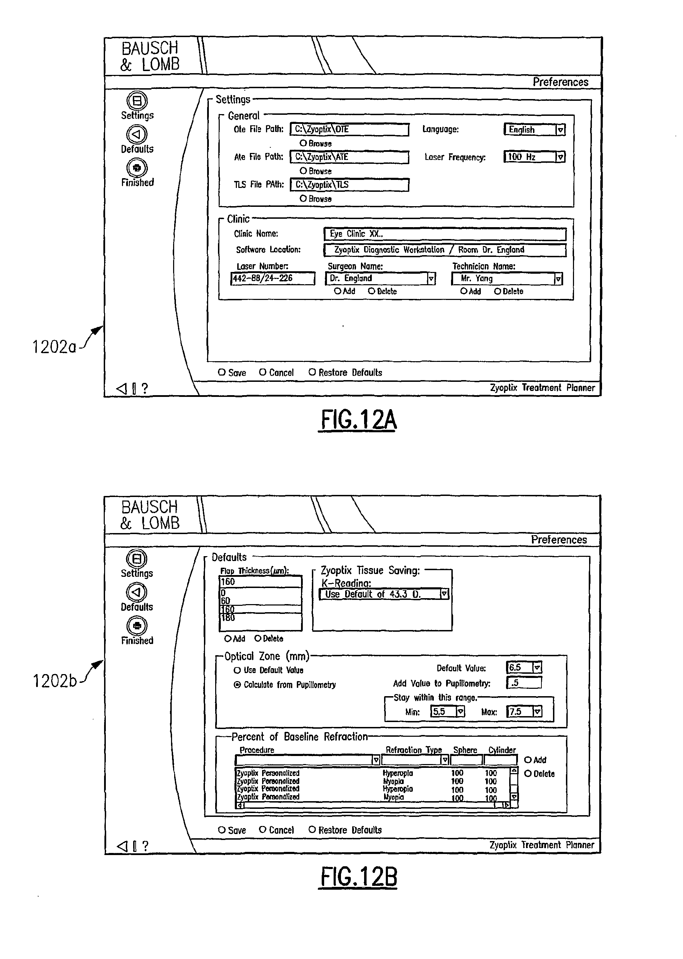

Illustrative display screens 1202a, 1202b of the aforementioned Preference settings are shown in Figs. 12a and 12b, which correspond, respectively, to the displays 314, 316 illustrated in Fig. 8 and Fig. 10 for an exemplary embodiment described earlier herein. The user then has the option to save the Preference settings and go on to the calculation phase of the Treatment Planner program, or return to the main menu.

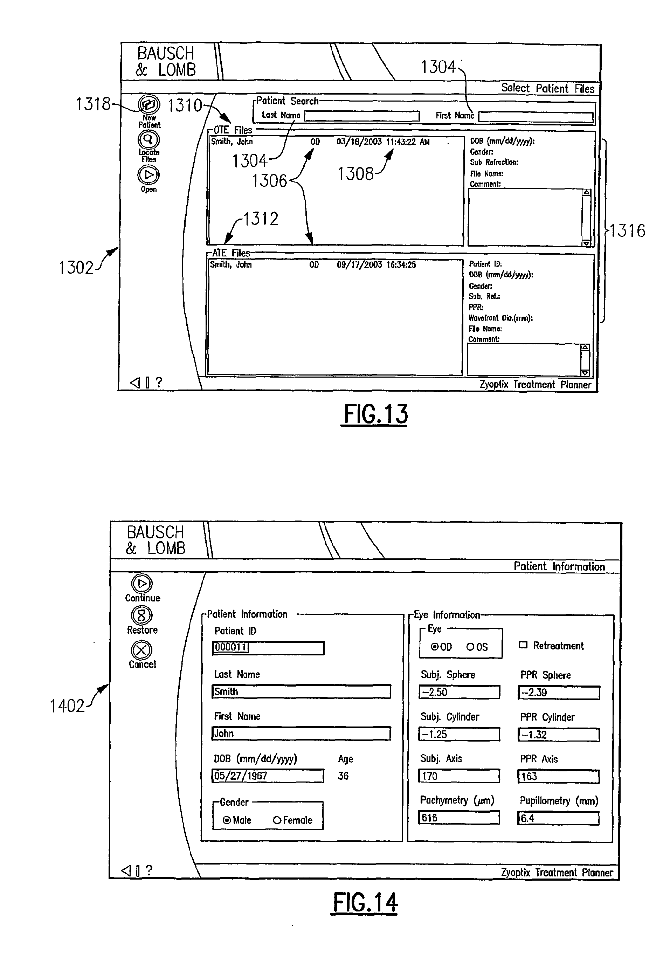

According to this exemplary embodiment, patient selection and treatment calculation is begun by activating the "Select Patient" icon 3004 on the main menu screen 312 shown in Fig. 6. When the Select Patient icon is activated, a display screen 1302 as shown in Fig. 13 is presented. The diagnostic files located in the folders specified in the Preference section, described above, are displayed, sorted by .OTE (corneal diagnostic data) files 1310 and .ATE (wavefront diagnostic) files 1312 separately. As shown, the displayed data includes windows 1304 for the patient's last and first names, a window 1306 for the eye (OS or OD) in question, and a window 1308 for the date and time of data acquisition. It will be appreciated that other arrangements and content of stored and displayed information may be presented depending upon a variety of factors known to those skilled in the art. In an exemplary aspect, selection of the .OTE or .ATE file will present additional data 1316 in each file that may help to clearly identify a patient or a specific measurement; for example, date of birth, refractive values, file name, etc. In another aspect, specific files can be searched by using the patient search area 1304 shown on the display by entering the name of a patient whose data has previously been saved or, in another example, by scrolling through a list of patient names and selecting the desired patient.

In an alternative aspect, a user may wish to directly calculate a treatment based on subjective refraction in which case neither the .ATE nor the .OTE files will be used. A "New Patient" icon 1318 can then be selected, which brings up a patient information screen 1402 as illustrated in Fig. 14. Basic patient information can be viewed and verified, modified or entered in the patient information screen 1402. In an exemplary aspect, the following data are displayed in the patient information window: patient ID; patient last name; patient first name; date of birth; gender; eye (OD/OS); re-treatment; subjective refraction (sphere, cylinder, axis); PPR (Predicted Phoropter Refraction; i.e., objective manifest refraction values based on .ATE file data; see application serial number 10/100782 entitled OBJECTIVE MEASUREMENT OF EYE REFRACTION filed on March 18, 2002);; pachymetry (from .OTE file or based on an ultrasound diagnostic measurement or other suitable diagnostic measurement); and pupillometry. In an exemplary aspect, the allowed pupil diameter range is 4mm to 11mm. In an exemplary aspect, input values that have been modified may appear in a different color or be otherwise distinguished for screen viewing. The user may then verify the data and continue on to treatment calculation or return to a previous screen for data re-entry or cancellation. In an exemplary aspect, a warning message (not shown) will be displayed if a required entry or an input value is outside of a predetermined default range.

According to the instant exemplary embodiment, two treatment options are available to the user. They are referred to herein as the Zyoptix Personalized Treatment algorithm and the Zyoptix Tissue Saving algorithm. The Zyoptix Personalized Treatment calculation option is a wavefront optimization treatment based on wavefront information data (.ATE files) and corneal topography data (.OTE files). In an exemplary aspect, the Personalized Treatment mode supports calculation of myopic and hyperopic treatments. According to this aspect, the sphere and cylinder of treatment refraction are user modifiable variables. The treatment refraction is expressed in a percentage of the PPR. The percent value is a user modifiable variable. The optical zone (OZ) of the treatment is also a user modifiable variable. The Personalized Treatment option algorithm is based upon the optimized placement of 2mm and 1mm laser spots on the corneal stroma provided, for example, by the Technolas 217z laser system. Treatment is centered over the pupil center. In this aspect, the Personalized Treatment option supports iris recognition parameters such as cyclotortion and pupil center shift, for example.

The Zyoptix Tissue Saving treatment calculation is based on measurements of subjective refraction, keratometric values (K-readings) and optical zone. In an exemplary aspect, this treatment option uses a combination of 2mm and 1mm truncated Gaussian beams for ablation, and has demonstrated tissue saving for myopic treatments compared to Planoscan treatments, which use a 2mm hard top beam profile, as those skilled in the art will understand. An amount of tissue saving may also be achieved over the Personalized Treatment option described above due to the fact that small deformations of the optical system, known as higher order aberrations, are not attempted to be corrected in this mode. Compared to the Planoscan algorithm, which assumes an average normalized shape for the preoperative cornea (K=43.3D), the actual curvature value for the corneal steepness (K-reading) can be included in the Tissue Saving treatment mode either by importing the value form the .OTE file, entering a K-value, or using a default value K= 43.3D. Treatment in this mode may be centered at the pupil center or, alternatively, toward the Purkinje reflex at the discretion of the surgeon. In the exemplary embodiment, the .ATE file has no influence on this treatment mode. In a related alternative aspect, an aspheric tissue saving treatment could be selected by using selected pre- and post-operative aspheric corneal shape factors, Q, Q'. In this aspect, conical surface could be determined according to the equation:

$$Z = \frac{p^2}{R + \sqrt{[R^2 - (1+Q)p^2]}}$$

Where
Z is the sag of the conical surface,

$$p^2 = x^2 + y^2,$$

R = central radius of curvature, and
$-1 \le Q \le 1$ ($Q \ne 0$), where the surface can be a prolate or oblate ellipsoid, a parabola, or a hyperbola. In an aspect of this embodiment, the conic constants Q (and Q') define biconic surfaces; i.e., Q (and Q') and the central radius of curvature, R (and R'), are functions of x, y, and may be different in the x and y directions. A biconic surface allows specification of $R_x$, Ry, $Q_x$, $Q_y$ (as well as their respective post-operative values) directly. As those skilled in the art will understand, the sag, Z, of a biconic can be expressed as

$$Z = \frac{c_x x^2 + c_y y^2}{1 + \sqrt{[1-(1+Q_x)c_x^2 x^2 - (1+Q_y)\, c_y^2 y^2]}}$$

where

$$c_z^{\text{biconic}} = \frac{-s_x c_x x^2 - s_y c_y y^2}{c_x x^2 + c_y y^2}$$

and

$$R_z^{\text{biconic}} = \frac{+\, c_x x^2 + c_y y^2}{-\, s_x c_x x^2 - s_y c_y y^2}$$

and

$$s_x = -(1+Q_x)\,, \quad s_y = -\,(1+Q_y)$$

so that

$$-z^2 c_z^{\text{biconic}} = -2z + (c_x x^2 + c_y y^2).$$

Substituting

$$z = z' + R_z^{\text{biconic}}$$

then,

$$c_x x^2 + c_y y^2 + c_z^{\text{biconic}} z'^2 = 1/ c_z^{\text{biconic}}$$

and since

$$c_x = 1/R_x, \quad c_y = 1/R_y,$$

then

$$x^2/R_x + y^2/R_y + z'^2/ R_z^{\text{biconic}} = R_z^{\text{biconic}}.$$

Employing the definitions

$$c_z = \frac{-s_x c_x - s_y c_y}{2}$$

and

$$\Delta = \frac{-s_x c_x + s_y c_y}{2}$$

gives (in series expanded form)

$$
\begin{aligned}
c_z^{\text{biconic}} &= \frac{- s_x c_x^2 x^2 - s_y c_y^2 y^2}{c_x x^2 + c_y y^2} \\
&= \frac{c_x(c_z + \Delta)x^2 + c_y(c_z - \Delta)y^2}{c_x x^2 + c_y y^2} \\
&= c_z - \frac{\Delta( c_x x^2 - c_y y^2)}{c_x x^2 + c_y y^2}.
\end{aligned}
$$

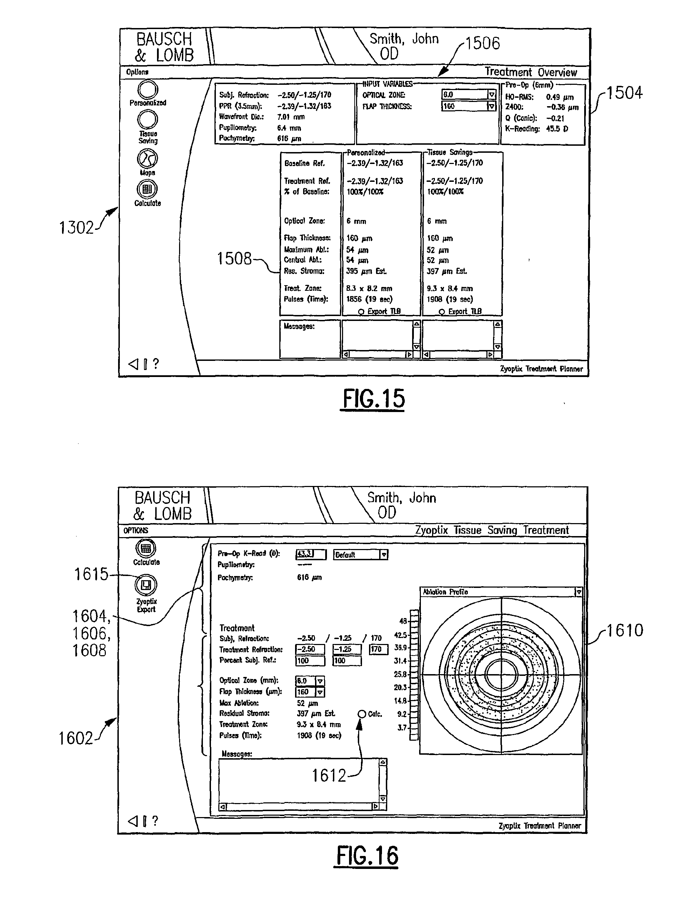

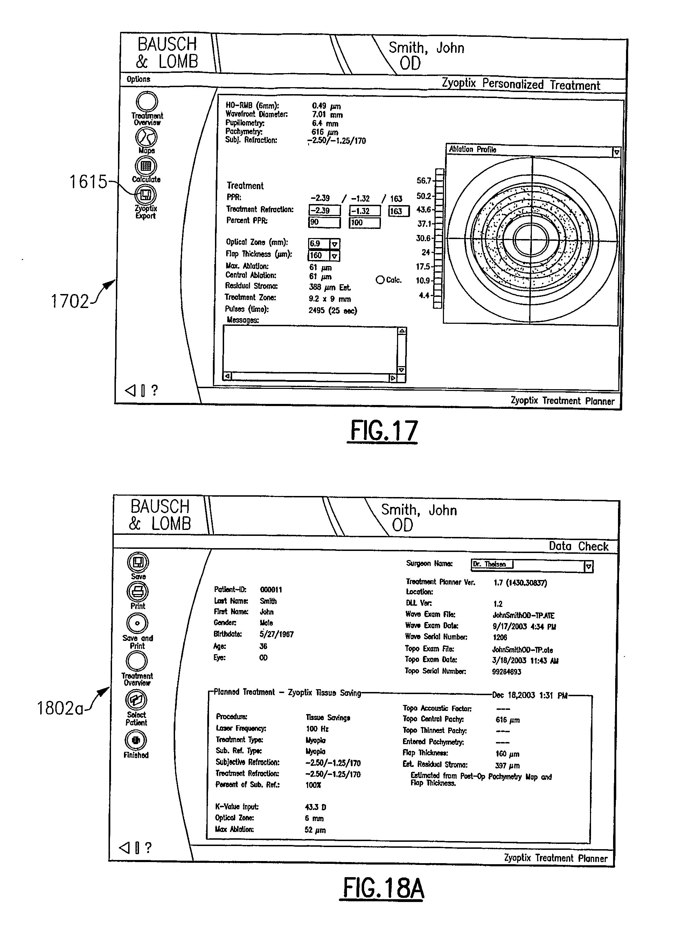

Depending on the treatment parameters, three different options of a treatment calculation screen may be presented to the user in an exemplary aspect. First, a treatment overview screen 1502 as shown in Fig. 15 is presented if both .OTE and .ATE files have been selected and more than one treatment option is available. From the treatment overview screen 1502, the user can either go straight to the calculation screen for one of the available treatment modes, or the user can perform a simultaneous calculation of the two displayed treatment modes, which allows a direct comparison of the results as displayed in the treatment overview window. As shown on screen 1502, the display parameters 1504 include (in addition to what is described in the individual calculation screens, described below) Z400 (Zernike spherical aberration coefficient) at 6mm, the conic constant, Q, at 6mm pupil diameter derived from the topography data, and a mean K-reading at 6mm pupil diameter derived from the topography data of the .OTE file. Input parameters 1506 include optical zone and flap thickness. Calculated output parameters 1508 include maximum ablation, central ablation, residual stroma (estimated or calculated), treatment zone, amount of pulses and treatment time. Alternatively, a Tissue Saving calculation screen 1602 as shown in Fig. 16 will display if only the .OTE file was selected, if neither the .OTE nor the .ATE files were selected, or if the .OTE and the .ATE files were selected but the personalized treatment mode is not available for the selected patient. The third alternative option is the display of a Personalized calculation screen 1702 as show in Fig. 17. This screen is displayed if the Personalized mode is selected or if the Tissue Saving mode is not available for the selected patient.

As illustrated in Fig. 16, the tissue saving calculation screen 1602 includes display parameters 1604 relating to pachymetry, pupillometry, and subjective refraction; input parameters 1606 include K-reading, treatment refraction, percent of subjective refraction, optical zone and flap thickness. Primary output parameters 1608 include maximum ablation, estimated residual stroma, treatment zone, number of pulses and treatment time. In various aspects, the estimated residual stroma thickness is based on an entered pachymetry value minus an entered flap thickness value minus a calculated maximum ablation value; alternatively, an estimated residual stroma value is based on pachymetry map data, entered flap thickness and the calculated ablation profile; alternatively, a more accurate (albeit, more time consuming) calculation of residual stroma can be selected if desired. A calculation icon 1612 is provided close to the estimated residual stroma value if the user wishes to verify the calculated residual stroma thickness.

As further shown in Fig. 16, an ablation profile map 1610 is displayed on the right hand side of the screen 1602. In an aspect, a map field is provided for accessing a pull-down list of the available maps that can be viewed. For example, if no .OTE files were loaded, no pre-op maps will be available; if a .OTE file was loaded, pre-operative maps including axial keratometric maps, anterior elevations maps, posterior elevation maps and pachymetry maps will be available. Further, if a .OTE file was loaded, simulated post-op pachymetry maps will be available. In the exemplary aspect, the map of the ablation profile is shown by default after calculation of a treatment.

Fig. 17 shows a Personalized Treatment mode calculation screen 1702. For this treatment mode, the display parameters 1704 include pachymetry, pupillometry, higher order (RMS) aberration values at a 6mm pupil diameter, wavefront diameter, subjective refraction and PPR. Input parameters 1706 include treatment refraction, percent of PPR, optical zone and flap thickness. Calculated output parameters 1708 include maximum ablation, central ablation, residual stroma (estimated or calculated as described above), treatment zone, amount of pulses and treatment time. A pull-down list of maps is, again, available. For example, pre-op maps include axial keratometric map, anterior elevation map, posterior elevation map, pachymetry map, higher order wavefront map and total wavefront map. Simulated post-op anterior elevation, axial keratometric, and pachymetry maps are also available, as well as the default ablation profile map 1710 as shown.

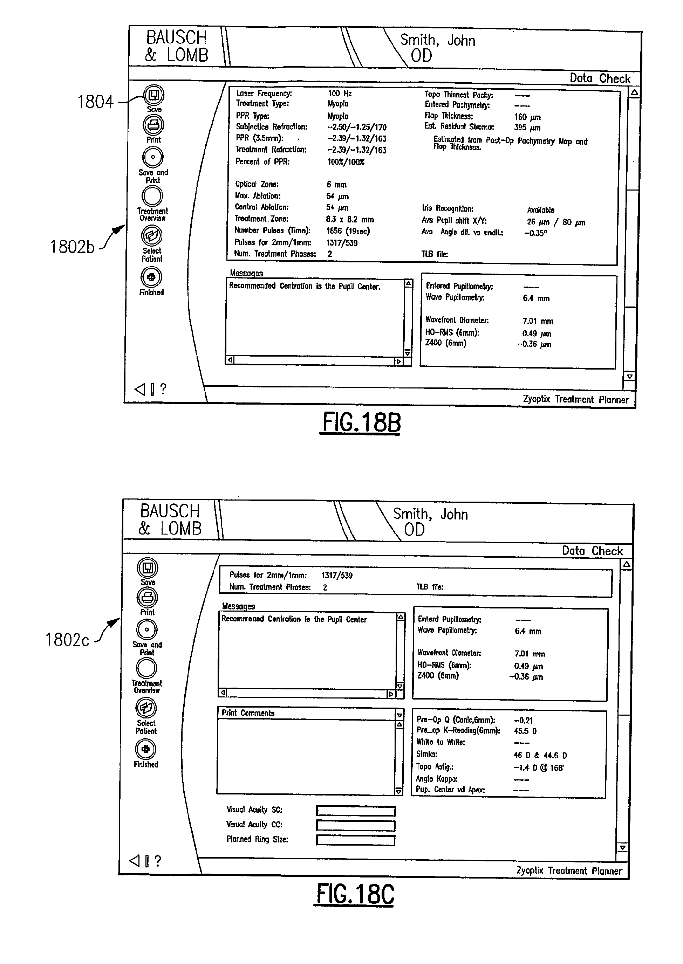

From any of the calculation screens the user may select an export icon 1615 that then brings up a Data Check screen 1802a,b,c as shown in Figs. 18a,b,c, respectively. The Data Check screen 1802, corresponding to a similar screen display 315a,b shown in Figs. 9a,b, for a previously described exemplary embodiment, allows the user to re-check and print the screen information before saving the .TLS (treatment) file. In addition to parameters shown either in the patient information screen or the individual calculations screens, other relevant information is displayed in the data check screen including: amount of treatment phases, number of pulses for 2mm and 1mm beams, iris recognition data if available for the particular treatment, type of algorithm (myopia, hyperopia, mixed astigmatism) used as the basis for the calculation, refraction, type of baseline refraction, and additional information from the topography and wavefront diagnostics.

When the user is ultimately satisfied with the data and intends to perform a treatment, clicking on a Save icon 1804 creates the .TLS file, which will be saved under the file path chosen in the Preferences described herein above.

Table 1 of Fig. 19 shows an exemplary range of parameter values accepted by the software according to the exemplary embodiment of the invention.

Notwithstanding the embodiments specifically illustrated and described herein, it will be appreciated by those skilled in the art that various modifications and variations of the instant invention are possible in light of the description set forth above and the appended claims.

**Claims**

**1.** A system used for planning a treatment for vision correction in a patient's eye, comprising:

means (310) for receiving a diagnostic input data (210) about the patient's vision, wherein the diagnostic input data (210) comprises at least one of wavefront data only, wavefront and topography data with or without corneal pachymetry data, and one of the preceding data plus other selected algorithm influencing data, for analyzing the input data (210), for parameterizing the input data adapted to automatically classify the patient's eye into one of a predetermined plurality of classification sets (135, 140, 145, 150) to automatically select a plurality of potentially useable treatment algorithms from a database comprising an equal or larger number of available treatment algorithms (265) comprising at least a standard ablation treatment, a customized or semi-customized wavefront-based treatment and a topographically-based treatment or a hybrid-driven treatment based upon the classification, wherein the analyzing means is configured to identify an allowable limit parameter for each of the available treatment algorithms (265) and to determine the potentially useable treatment algorithms based upon whether the allowable limit parameters are exceeded, and for processing said potentially useable treatment algorithms based upon the input data (210) and one or more algorithm default parameters;

means (370) for displaying a plurality of treatment plans (290) corresponding, respectively, to the plurality of potentially useable treatment algorithms; for selectively modifying the algorithm default parameters and other defined treatment influencing parameters, and for displaying a respective plurality of modified treatment plans, operatively connected to said receiving means.

2. The system of claim 1, wherein the means for parameterizing and classifying is capable of determining whether the eye is one of a) a virgin eye or a previously treated eye, b) a regular eye or an irregular eye, and c) a myopic eye with or without mixed astigmatism or a hyperopic eye with or without mixed astigmatism.

3. The system of claim 1, wherein the input data (210) types include diagnostic wavefront data and diagnostic corneal data, the data of each of which is stored in a different, user selectable file.

4. The system of claim 1, wherein the display means (370) is further adapted for selecting a preferred treatment plan.

5. The system of claim 4, further comprising a device readable storage medium (282) that can selectively store the plurality of modified treatment plans including the selected preferred treatment plan.

6. The system of claim 5, further comprising a therapeutic laser ablation component (294) in operative communication with said storage medium (282) and adapted to apply the selected preferred treatment plan to the patient's eye.

7. The system of claim 4, wherein each one of the plurality of modified treatment plans includes a respective instruction describing a laser ablation shot file.

8. The system of claim 7, wherein the laser ablation shot files comprise at least a placement and sequence determination of laser ablation shots on the patient's eye.

9. The system of claim 1, wherein the larger number of available treatment algorithms (265) is selected from a group of at least two relating to a myopia treatment only, a hyperopia treatment only, a myopia treatment with astigmatism, a hyperopia treatment with astigmatism, a lower-order aberration correction treatment, a higher-order aberration correction treatment, a re-treatment, a spherical corrective treatment, an aspherical corrective treatment, a LASIK treatment, a LASEK treatment, a PRK treatment, a nomogram adjusted treatment, and a customized treatment.

10. The system of claim 1, wherein the receiving means (310) comprises a software-driven calculation module.

11. The system of claim 1, wherein the display means (370) comprises a display device displaying a multi-level GUI.

12. The system of claim 4, wherein the device readable storage medium (282) comprises one of a floppy disk, a CD, a DVD, a computer hard drive, and electromagnetic data storage means.

13. The system of claim 5, wherein the therapeutic laser ablation component (294) comprises a laser system adapted for photoablation of corneal tissue operatively connected to an eye tracker component.

14. The system of claim 1, wherein the receiving (310) and display means (370), respectively, are further adapted for sorting and displaying a plurality of user defined criteria for each of the calculated treatment plans.

15. The system of claim 11, wherein the multi-level GUI includes a data check display showing summary data of the selected treatment plan.

16. The system of claim 11, wherein the multi-level GUI includes a start-up navigation screen.

17. The system of claim 16, further including a screen for viewing user modifiable preference and default settings.

18. The system of claim 16, further including a screen for viewing a diagnostic data file.

19. The system of claim 16, further including a screen for viewing patient information.

20. The system of claim 16, further including a screen for viewing a treatment plan calculation.

21. The system of claim 20, further including a screen for simultaneously viewing at least two treatment plan calculations.

22. The system of claim 16 further including a data check screen.

23. A method for aiding the selection of a treatment plan for correcting vision in a patient's eye, comprising:

obtaining selected diagnostic input data (210) about the patient's eye, wherein the diagnostic input data comprises at least one of wavefront data only, wavefront and topography data with or without corneal pachymetry data, and one of the preceding data plus other selected data,

analyzing (230) the input data (210), parameterizing the input data to classify the patient's eye into one of a predetermined plurality of classification sets (135, 140, 145, 150) to automatically select a plurality of potentially useable treatment algorithms from a database comprising an equal or larger number of available treatment algorithms (265) comprising at least a standard ablation treatment, a customized or semi-customized wavefront-based treatment and a topographically-based treatment or a hybrid-driven treatment based upon the classification wherein the analyzing step comprises identifying an allowable limit parameter for each of the available treatment algorithms (265) and determining the potentially useable treatment algorithms based upon whether the allowable limit parameters are exceeded, and processing said plurality of potentially useable treatment algorithms, wherein said available treatment algorithms utilize one or more default parameters;

presenting (270) for review a plurality of treatment plans (290) corresponding to said plurality of potentially useable treatment algorithms;

selectively modifying the one or more default parameters and other treatment parameters;

re-processing (230') said plurality of potentially useable treatment algorithms using the modified parameters; and

re-presenting (270') for further review the plurality of treatment plans corresponding to said plurality of potentially useable treatment algorithms.

24. The method of claim 23, wherein the classifying step comprises determining whether the eye is one of a) a virgin eye or a previously treated eye, b) a regular eye or an irregular eye, and c) a myopic eye with or without mixed astigmatism or a hyperopic eye with or without mixed astigmatism.

25. The method of claim 23, wherein each of the selected input data types is stored in a different, user selectable file.

26. The method of claim 23, further comprising selecting (292) one of the treatment plans.

27. The method of claim 23, wherein said treatment plans comprise data relating to at least some of a laser ablation spot size, a laser ablation shot placement, a laser ablation shot sequence, a laser shot file, a simulated post-operative wavefront map, a simulated post-operative topography map, a simulated ablation profile, an axial keratometric map, corneal pachymetry, optical zone dimension, a manifest refraction value, a target refraction value, higher-order aberration information, a residual stromal tissue depth, and a vision metric.

28. The method of claim 27, further comprising selectively sorting at least some of the data for each of the treatment plans according to a user preferred criteria including at least one of the target refraction, the residual stromal depth, and the optical zone dimension.

29. The method of claim 28, further comprising optimizing at least one of the user preferred criteria.

30. The method of claim 28, further comprising selectively presenting the sorted data for review by the user.

31. The method of claim 23, wherein said available treatment algorithms (265) comprise at least two algorithms selected from a group including a myopia treatment only, a hyperopia treatment only, a myopia treatment with astigmatism, a hyperopia treatment with astigmatism, a lower-order aberration correction treatment, a higher-order aberration correction treatment, a higher-order corneal aberration treatment, a re-treatment, a spherical corrective treatment, an aspherical corrective treatment, a LASIK treatment, a LASEK treatment, a PRK treatment, a nomogram adjusted treatment, and a customized treatment.

32. The method of claim 23, wherein said one or more default parameters represent a value for parameters including at least one of an optical zone, a corneal flap thickness, and another parameter that influences the calculation of the algorithms.

33. The method of claim 23, further comprising providing a display device (370) displaying a graphical user interface (GUI) for use by a user.

34. The method of claim 23, further comprising saving (280) the plurality of reprocessed treatment plans on a device readable storage medium (282).

35. The method of claim 23, wherein the sorting step comprises optimizing at least one of the user preferred criteria and sorting based upon said optimization.

36. The method of claim 33, wherein displaying said GUI further comprises selectively displaying a data check screen that contains summary data of the selected treatment plan.

37. The method of claim 35, wherein the summary data includes one or more of patient identifying information, selected treatment plan, manifest refraction, objective refraction for a given pupil diameter, pre- and targeted post-operative K values, pre- and targeted post-operative Q values, optical zone size, treatment zone, number of ablation shots and treatment time, maximum ablation depth, central ablation depth, and residual stromal pachymetry for a specified corneal flap thickness.

38. The method of claim 23, wherein the processing step further comprises utilizing at least one of rotational eye-tracking data and microkeratometric data.

39. The method of claim 23, further comprising automatically recommending to the user a plurality of preferred treatment plans.

40. The method of claim 33, wherein the use of the GUI comprises user options selected from a group comprising patient selection, default value adjustment, displaying processing software information, inputting patient data, and creating display screen headers.

41. The method of claim 33, wherein the display of the GUI includes color coding of the treatment plans, selective display of rotational eye-tracking information, selective display of microkeratometric information, minimization/maximization of data presentation size, a warning message based on a user selected parameter modification, saving of treatment plan parameters to a selected storage medium, and other parametric monitoring.

42. The method of claim 23, wherein the plurality of treatment plans includes a customized treatment plan that reduces higher order wavefront aberrations and a non-customized treatment plan that improves lower order aberrations.

43. The method of claim 42, wherein the non-customized treatment plan is based, at least in part, on a non-normalized K-reading value of the patient's eye.

44. The method of claim 42, wherein the non-customized treatment plan is based, at least in part, on an aspherical corneal shape factor, Q, of the patient's eye.

45. The method of claim 42, wherein each of the treatment plans is based, at least in part, on a prospective residual stroma thickness.

46. The method of claim 45, wherein the prospective residual stroma thickness value is an estimated value.

47. The method of claim 45, wherein the prospective residual stroma thickness value is a calculated value.

**Patentansprüche**

1. System, das verwendet wird, um eine Behandlung zur Sehkorrektur in einem Auge eines Patienten zu planen, das aufweist:

eine Einrichtung (310) zum Empfangen von diagnostischen Eingabedaten (210) über das Sehvermögen des Patienten, wobei die diagnostischen Eingabedaten (210) nur Wellenfrontdaten und/oder Wellenfront- und Topographiedaten mit oder ohne Hornhautdickenmessdaten und/oder eines der vorhergehenden Daten plus andere ausgewählte den Algorithmus beeinflussende Daten aufweisen, um die Eingabedaten (210) zu analysieren, um die Eingabedaten zu parametrisieren, die geeignet sind, um das Auge des Patienten automatisch in eine von mehreren vorgegebenen Klassifikationsgruppen (135, 140, 145, 150) zu klassifizieren, um automatisch mehrere potentiell verwendbare Behandlungsalgorithmen aus einer Datenbank auszuwählen, die eine gleiche oder größere Anzahl verfügbarer Behandlungsalgorithmen (265) aufweist, die wenigstens eine Standardablationsbehandlung, eine kundenspezifische oder halbkundenspezifische Wellenfront-basierte Behandlung und eine topographisch basierte Behandlung oder eine hybridgetriebene Behandlung basierend auf der Klassifikation aufweist, wobei die Analyseeinrichtung konfiguriert ist, um einen zulässigen Grenzparameter für jeden der verfügbaren Behandlungsalgorithmen (265) zu identifizieren und die potentiell verwendbaren Behandlungsalgorithmen basierend darauf zu bestimmen, ob die zulässigen Grenzparameter überschritten sind, und um die potentiell verwendbaren Behandlungsalgorithmen basierend auf den Eingabedaten (210) und einem oder mehreren Algorithmusvorgabewerten zu verarbeiten;

eine Einrichtung (370) zum Anzeigen mehrerer Behandlungspläne (290), die jeweils den mehreren potentiell verwendbaren Behandlungsalgorithmen entsprechen, um die Algorithmusvorgabeparameter und andere definierte behandlungsbeeinflussende Parameter zu modifizieren und um jeweilige mehrere modifizierte Behandlungspläne anzuzeigen, die jeweils mit der Empfangseinrichtung betriebsfähig verbunden sind.

2. System nach Anspruch 1, wobei die Einrichtung zum Parametrisieren und Klassifizieren fähig ist, zu bestimmen, ob das Auge a) ein unberührtes Auge oder ein früher behandeltes Auge ist oder b) ein reguläres oder irreguläres Auge ist oder c) ein kurzsichtiges Auge mit oder ohne gemischtem Astigmatismus oder ein weitsichtiges Auge mit oder ohne gemischten Astigmatismus ist.

3. System nach Anspruch 1, wobei die Typen der Eingabedaten (210) diagnostische Wellenfrontdaten und diagnostische Hornhautdaten umfassen, wobei die Daten jeweils in einer verschiedenen vom Nutzer auswählbaren Datei gespeichert werden.

4. System nach Anspruch 1, wobei die Anzeigeeinrichtung (370) ferner geeignet ist, um einen bevorzugten Behandlungsplan auszuwählen.

5. System nach Anspruch 4, das ferner ein maschinenlesbares Speichermedium (282) aufweist, das die mehreren modifizierten Behandlungspläne einschließlich des ausgewählten bevorzugten Behandlungsplans selektiv speichern kann.

6. System nach Anspruch 5, das ferner eine Laserablationstherapiekomponente (294) in betriebsfähiger Verbindung mit dem Speichermedium (282) aufweist und geeignet ist, den ausgewählten bevorzugten Behandlungsplan auf das Auge eines Patienten anzuwenden.

7. System nach Anspruch 4, wobei jeder einzelne der mehreren modifizierten Behandlungspläne eine jeweilige Anweisung umfasst, die eine Laserablationsschussdatei beschreibt.

8. System nach Anspruch 7, wobei die Laserablationsschussdateien wenigstens eine Anordnungs- und Abfolgebestimmung von Laserablationsschüssen auf das Auge des Patienten aufweist.

9. System nach Anspruch 1, wobei die größere Anzahl verfügbarer Behandlungsalgorithmen (265) aus einer Gruppe von wenigstens zwei ausgewählt wird, die nur eine Kurzsichtigkeitsbehandlung, nur eine Weitsichtigkeitsbehandlung, eine Kurzsichtigkeitsbehandlung mit Astigmatismus, eine Weitsichtigkeitsbehandlung mit Astigmatismus, eine Aberrationskorrekturbehandlung niedrigerer Ordnung, eine Aberrationskorrekturbehandlung höherer Ordnung, eine Nachbehandlung, eine sphärische Korrekturbehandlung, eine asphärische Korrekturbehandlung, eine LASIK-Behandlung, eine LASEK-Behandlung, eine PRK-Behandlung, eine mit Nomogramm eingestellte Behandlung und eine kundenspezifische Behandlung betreffen.

10. System nach Anspruch 1, wobei die Empfangseinrichtung (310) ein softwaregetriebenes Berechnungsmodul aufweist.

11. System nach Anspruch 1, wobei die Anzeigeeinrichtung (370) eine Anzeigevorrichtung, die eine mehrstufige GUI anzeigt, aufweist.

12. System nach Anspruch 4, wobei das maschinenlesbare Speichermedium (282) eine Diskette, eine CD, eine DVD, ein Computerfestplattenlaufwerk oder eine elektromagnetische Datenspeichereinrichtung aufweist.

13. System nach Anspruch 5, wobei die Laserablationskomponente (294) ein Lasersystem aufweist, das für die Photoablation von Hornhautgewebe geeignet ist, das mit einer Eye-Tracker-Komponente verbunden ist.

14. System nach Anspruch 1, wobei die Empfangs- (310) und Anzeigeeinrichtung (370) jeweils ferner geeignet sind, mehrere nutzerdefinierte Kriterien für jeden der berechneten Behandlungspläne zu sortieren und anzuzeigen.

15. System nach Anspruch 11, wobei die mehrstufige GUI eine Datenprüfanzeige umfasst, die zusammengefasste Daten des ausgewählten Behandlungsplans zeigt.

16. System nach Anspruch 11, wobei die mehrstufige GUI einen Navigationsstartbildschirm umfasst.

17. System nach Anspruch 16, das ferner einen Bildschirm zum Betrachten von durch einen Benutzer modifizierbaren Vorzugs- und Vorgabeeinstellungen umfasst.

18. System nach Anspruch 16, das ferner einen Bildschirm zum Betrachten einer Diagnosedatendatei umfasst.

19. System nach Anspruch 16, das ferner einen Bildschirm zum Betrachten von Patienteninformationen umfasst.

20. System nach Anspruch 16, das ferner einen Bildschirm zum Betrachten einer Behandlungsplanberechnung umfasst.

21. System nach Anspruch 20, das ferner einen Bildschirm zum gleichzeitigen Betrachten von wenigstens zwei Behandlungsplanberechnungen umfasst.

22. System nach Anspruch 16, das ferner einen Datenprüfbildschirm umfasst.

23. Verfahren zur Unterstützung der Auswahl eines Behandlungsplans zur Sehkorrektur in einem Auge eines Patienten, das aufweist:

Gewinnen von diagnostischen Eingabedaten (210) über das Auge des Patienten, wobei die diagnostischen Eingabedaten nur Wellenfrontdaten und/oder Wellenfront- und Topographiedaten mit oder ohne Hornhautdickenmessdaten und/oder eines der vorhergehenden Daten plus andere ausgewählte Daten aufweist;
Analysieren (230) der Eingabedaten (210), Parametrisieren der Eingabedaten, um das Auge des Patienten automatisch in eine von mehreren vorgegebenen Klassifikationsgruppen (135, 140, 145, 150) zu klassifizieren, um automatisch mehrere potentiell verwendbare Behandlungsalgorithmen aus einer Datenbank auszuwählen, die eine gleiche oder größere Anzahl verfügbarer Behandlungsalgorithmen (265) aufweist, die wenigstens eine Standardablationsbehandlung, eine kundenspezifische oder halbkundenspezifische Wellenfront-basierte Behandlung und eine topographisch basierte Behandlung oder eine hybridgetriebene Behandlung basierend auf der Klassifikation aufweist, wobei der Analyseschritt das Identifizieren eines zulässigen Grenzparameters für jeden der verfügbaren Behandlungsalgorithmen (265) und das Bestimmen der potentiell verwendbaren Behandlungsalgorithmen basierend darauf umfasst, ob die zulässigen Grenzparameter überschritten sind, und das Verarbeiten der potentiell verwendbaren Behandlungsalgorithmen, wobei die verfügbaren Behandlungsalgorithmen einen oder mehrere Vorgabeparameter nutzen;
Darstellen (270) zur Durchsicht mehrerer Behandlungspläne (290), die den mehreren potentiell verwendbaren Behandlungsalgorithmen entsprechen;
selektives Modifizieren des einen oder der mehreren Vorgabeparameter und anderer Behandlungsparameter;
Wiederverarbeiten (230') der mehreren potentiell verwendbaren Behandlungsalgorithmen unter Verwendung der modifizierten Parameter; und
Wiederdarstellen (270') zur weiteren Durchsicht der mehreren Behandlungspläne, die den mehreren potentiell verwendbaren Behandlungsalgorithmen entsprechen.

24. Verfahren nach Anspruch 23, wobei der Klassifizierungsschritt die Bestimmung aufweist, ob das Auge a) ein unberührtes Auge oder ein früher behandeltes Auge ist oder b) ein reguläres oder irreguläres Auge ist oder c) ein kurzsichtiges Auge mit oder ohne gemischtem Astigmatismus oder ein weitsichtiges Auge mit oder ohne gemischten Astigmatismus ist.

25. Verfahren nach Anspruch 23, wobei jeder der ausgewählten Eingabedatentypen in einer verschiedenen vom Nutzer auswählbaren Datei gespeichert wird.

26. Verfahren nach Anspruch 23, das ferner das Auswählen (292) eines der Behandlungspläne aufweist.

27. Verfahren nach Anspruch 23, wobei die Behandlungspläne wenigstens Daten in Bezug auf einige der Folgenden aufweisen: die Laserablationspunktgröße, die Laserablationsschussanordnung, eine Laserablationsschussabfolge, eine Laserschussdatei, eine simulierte postoperative Wellenfrontabbildung, eine simulierte postoperative Topographiabbildung, ein simuliertes Ablationsprofil, eine axiale keratometrische Abbildung, Hornhautdickenmessung, optische Zonenabmessung, einen evidenten Brechungswert, einen Zielbrechungswert, eine Aberrationsinformation höherer Ordnung, eine Stromarestgewebetiefe und eine Sichtmetrik.

28. Verfahren nach Anspruch 27, das ferner das selektive Sortieren wenigstens einiger der Daten für jeden der Behandlungspläne gemäß einem nutzerbevorzugten Kriterium einschließlich der Zielbrechung und/oder der Stromaresttiefe und/oder der optischen Zonenabmessung aufweist.

29. Verfahren nach Anspruch 28, das ferner das Optimieren wenigstens eines der nutzerbevorzugten Kriterien aufweist.

30. Verfahren nach Anspruch 28, das ferner das selektive Vorstellen der sortierten Daten für die Durchsicht durch den Nutzer aufweist.

31. Verfahren nach Anspruch 23, wobei die verfügbaren Behandlungsalgorithmen (265) wenigstens zwei Algorithmen aufweisen, die aus einer Gruppe ausgewählt werden, die umfasst: nur eine Kurzsichtigkeitsbehandlung, nur eine Weitsichtigkeitsbehandlung, eine Kurzsichtigkeitsbehandlung mit Astigmatismus, eine Weitsichtigkeitsbehandlung mit Astigmatismus, eine Aberrationskorrekturbehandlung niedrigerer Ordnung, eine Aberrationskorrekturbehandlung höherer Ordnung, eine Hornhautaberrationsbehandlung höherer Ordnung, eine Nachbehandlung, eine sphärische Korrekturbehandlung, eine asphärische Korrekturbehandlung, eine LASIK-Behandlung, eine LASEK-Behandlung, eine PRK-Behandlung, eine mit Nomogramm eingestellte Behandlung und eine kundenspezifische Behandlung.

32. System nach Anspruch 23, wobei der eine oder die mehreren Vorgabeparameter einen Wert für Parameter darstellen, der eine optische Zone und/oder eine Hornhautscheibchendicke und/oder einen anderen Parameter, der die Berechnung der Algorithmen beeinflusst, umfasst.

33. Verfahren nach Anspruch 23, das ferner das Bereitstellen einer Anzeigeeinrichtung (370), die eine graphische Benutzerschnittstelle (GUI) für die Verwendung durch einen Nutzer anzeigt, aufweist.

34. Verfahren nach Anspruch 23, das ferner das Speichern (280) der mehreren wiederverarbeiteten Behandlungspläne auf einem maschinenlesbaren Speichermedium (282) aufweist.

35. Verfahren nach Anspruch 23, wobei der Sortierschritt das Optimieren wenigstens eines der nutzerbevorzugten Kriterien und das Sortieren basierend auf dieser Optimierung aufweist.

36. Verfahren nach Anspruch 33, wobei das Anzeigen der GUI ferner das selektive Anzeigen eines Datenprüfbildschirms aufweist, der zusammengefasste Daten des ausgewählten Behandlungsplans enthält.

37. Verfahren nach Anspruch 35, wobei die zusammengefassten Daten eines oder mehrere der folgenden umfassen: Patientenidentifizierungsinformationen, ausgewählten Behandlungsplan, evidente Brechung, objektive Brechung für einen gegebenen Pupillendurchmesser, prä- und Ziel-postoperative K-Werte, prä- und Ziel-postoperative Q-Werte, optische Zonengröße, Behandlungszone, Anzahl von Ablationsschüssen und Behandlungszeit, maximale Ablationstiefe, zentrale Ablationstiefe und Stromaresthornhautdickenmessung für eine spezifizierte Hornhautscheibchendicke.

38. Verfahren nach Anspruch 23, wobei der Verarbeitungsschritt ferner die Nutzung von Rotations-Eye-Tracking-Daten und/oder mikrokeratometrischer Daten aufweist.

39. Verfahren nach Anspruch 23, das ferner das automatische Empfehlen mehrerer bevorzugter Behandlungspläne an den Nutzer aufweist.

**40.** Verfahren nach Anspruch 33, wobei die Verwendung der GUI Nutzeroptionen aufweist, die aus einer Gruppe ausgewählt werden, die aufweist: Patientenauswahl, Vorgabewerteinstellung, Anzeigeverarbeitungssoftwareinformationen, Eingeben von Patientendaten und Erzeugen von Anzeigebildschirmtiteln.

**41.** Verfahren nach Anspruch 33, wobei die Anzeige der GUI die Farbkodierung der Behandlungspläne, die selektive Anzeige der Rotations-Eye-Tracking-Daten, die selektive Anzeige von mikrokeratometrischen Informationen, die Minimierung/Maximierung der Datendarstellungsgröße, eine Warnnachricht basierend auf einer nutzerausgewählten Parametermodifikation, das Speichern von Behandlungsplanparametern in ein ausgewähltes Speichermedium und sonstige Parameterüberwachung umfasst.

**42.** Verfahren nach Anspruch 23, wobei die mehreren Behandlungspläne einen kundenspezifischen Behandlungsplan, der Wellenfrontaberrationen höherer Ordnung verringert, und einen nicht kundenspezifischen Behandlungsplan, der Aberrationen niedrigerer Ordnung verbessert, umfassen.

**43.** Verfahren nach Anspruch 42, wobei der nicht kundenspezifische Behandlungsplan wenigstens teilweise auf einem nicht normierten K-Ablesewert des Auges des Patienten basiert.

**44.** Verfahren nach Anspruch 42, wobei der nicht kundenspezifische Behandlungsplan wenigstens teilweise auf einem asphärischen Hornhautformfaktor Q des Auges des Patienten basiert.

**45.** Verfahren nach Anspruch 42, wobei jeder der Behandlungspläne wenigstens teilweise auf einer voraussichtlichen Stromarestdicke basiert.

**46.** Verfahren nach Anspruch 45, wobei die voraussichtliche Stromarestdicke ein Schätzwert ist.

**47.** Verfahren nach Anspruch 45, wobei die voraussichtliche Stromarestdicke ein berechneter Wert ist.


**Revendications**

**1.** Système permettant de programmer un traitement de correction de la vision d'un oeil d'un patient, comprenant :

un moyen (310) pour la réception de données d'entrée diagnostiques (210) relatives à la vision du patient, où lesdites données d'entrée diagnostiques (210) comprennent au moins un des ensembles de données suivants : données de front d'onde seulement, données de front d'onde et de topographie avec ou sans données de pachymétrie cornéenne,
et l'un des ensembles de données susmentionné plus d'autres données d'influence d'algorithme sélectionnées, pour l'analyse des données d'entrée (210) afin de paramétrer les données d'entrée prévues pour classer automatiquement l'oeil du patient dans un ensemble d'une pluralité d'ensembles de classement (135, 140, 145, 150), pour la sélection automatique d'une pluralité d'algorithmes de traitement potentiellement exploitables d'une base de données comprenant un nombre égal ou supérieur d'algorithmes de traitement (265) disponibles, avec au moins un traitement d'ablation standard, un traitement personnalisé ou semi-personnalisé sur la base d'un front d'onde et un traitement sur une base topographique ou un traitement hybride sur la base du classement, le moyen d'analyse étant prévu pour identifier un paramètre de limite admissible pour chacun des algorithmes de traitement (265) disponibles et pour déterminer les algorithmes de traitement potentiellement exploitables en cas de dépassement des paramètres limites admissibles, et pour traiter lesdits algorithmes de traitement potentiellement exploitables sur la base des données d'entrée (210) et d'un ou de plusieurs paramètres d'algorithme par défaut ;
un moyen (370) pour afficher une pluralité de plans de traitement (290) correspondant respectivement à la pluralité d'algorithmes de traitement potentiellement exploitables,
pour la modification sélective des paramètres d'algorithme par défaut et d'autres paramètres influençant le traitement, et pour l'affichage d'une pluralité respective de plans de traitement modifiés, fonctionnellement reliés au moyen de réception.

**2.** Système selon la revendication 1, où le moyen de paramétrage et de classement est prévu pour déterminer si l'oeil est soit a) un oeil vierge ou un oeil précédemment traité, soit b) un oeil régulier ou un oeil irrégulier, soit c) un oeil myope avec ou sans astigmatisme mixte ou un oeil hypermétrope avec ou sans astigmatisme mixte.

3. Système selon la revendication 1, où les types de données d'entrée (210) comprennent des données de front d'onde de diagnostic et des données cornéennes de diagnostic, les données de chaque type étant stockées dans des fichiers différents, sélectionnables par l'utilisateur.

4. Système selon la revendication 1, où le moyen d'affichage (370) est en outre prévu pour sélectionner un plan de traitement préférentiel.

5. Système selon la revendication 4, comprenant en outre un support de stockage lisible par un dispositif (282) pouvant stocker sélectivement la pluralité des plans de traitement modifiés comprenant le plan de traitement préférentiel sélectionné.

6. Système selon la revendication 5, comprenant en outre un composant thérapeutique d'ablation par laser (294) en liaison fonctionnelle avec le support de stockage (282) et prévu pour appliquer le plan de traitement préférentiel sélectionné à l'oeil du patient.

7. Système selon la revendication 4, où chaque plan de la pluralité de plans de traitement modifiés comprend une instruction respective décrivant un fichier de tir d'ablation laser.

8. Système selon la revendication 7, où les fichiers de tir d'ablation laser comprennent au moins une détermination de positionnement et de séquence de tirs d'ablation laser sur l'oeil du patient.

9. Système selon la revendication 1, où le nombre supérieur d'algorithmes de traitement (265) disponibles est sélectionné dans un groupe d'au moins deux algorithmes relatifs à un traitement de la myopie seulement, un traitement de l'hypermétropie seulement, un traitement de la myopie avec astigmatisme, un traitement de l'hypermétropie avec astigmatisme, un traitement correctif d'aberration d'ordre inférieur, un traitement correctif d'aberration d'ordre supérieur, un retraitement, un traitement correctif sphérique, un traitement correctif asphérique, un traitement LASIK, un traitement LASEK, un traitement PRK, un traitement avec nomogramme d'ajustement, et un traitement personnalisé.

10. Système selon la revendication 1, où le moyen de réception (310) comprend un module de calcul assisté par logiciel.

11. Système selon la revendication 1, où le moyen d'affichage (370) comprend un dispositif d'affichage d'une interface graphique d'utilisateur à plusieurs niveaux.

12. Système selon la revendication 4, où le support de stockage lisible par un dispositif (282) comprend soit une disquette, soit un CD, soit un DVD, soit un disque dur d'ordinateur, soit un moyen de stockage électromagnétique.

13. Système selon la revendication 5, où le composant thérapeutique d'ablation par laser (294) comprend un système de laser prévu pour la photoablation d'un tissu cornéen, fonctionnellement relié à un composant de poursuite oculaire.

14. Système selon la revendication 1, où le moyen de réception (310) et le moyen d'affichage (370) sont en outre respectivement prévus pour trier et afficher une pluralité de critères définis par l'utilisateur pour chacun des plans de traitement calculés.

15. Système selon la revendication 11, où l'interface graphique d'utilisateur à plusieurs niveaux comprend un affichage de contrôle de données, affichant des données synthétiques du plan de traitement sélectionné.

16. Système selon la revendication 11, où l'interface graphique d'utilisateur à plusieurs niveaux comprend un écran de navigation de démarrage.

17. Système selon la revendication 16, comprenant en outre un écran pour la visualisation de réglages préférentiels modifiables par l'utilisateur et de réglages par défaut.

18. Système selon la revendication 16, comprenant en outre un écran pour la visualisation d'un fichier de données de diagnostic.

19. Système selon la revendication 16, comprenant en outre un écran pour la visualisation d'informations relatives au patient.

**20.** Système selon la revendication 16, comprenant en outre un écran pour la visualisation d'un calcul de plan de traitement.

**21.** Système selon la revendication 20, comprenant en outre un écran pour la visualisation simultanée d'au moins deux calculs de plan de traitement.

**22.** Système selon la revendication 16, comprenant en outre un écran de vérification de données.

**23.** Procédé d'aide à la sélection d'un plan de traitement de correction de la vision d'un oeil d'un patient, comprenant :

l'acquisition de données d'entrée diagnostiques (210) relatives à la vision du patient, lesdites données d'entrée diagnostiques comprenant au moins un des ensembles de données suivants : données de front d'onde seulement, données de front d'onde et de topographie avec ou sans données de pachymétrie cornéenne, et l'un des ensembles de données susmentionné plus d'autres données sélectionnées ;

l'analyse (230) des données d'entrée (210), le paramétrage des données d'entrée pour classer l'oeil du patient dans un ensemble d'une pluralité d'ensembles de classement (135, 140, 145, 150), pour la sélection automatique d'une pluralité d'algorithmes de traitement potentiellement exploitables d'une base de données comprenant un nombre égal ou supérieur d'algorithmes de traitement (265) disponibles, avec au moins un traitement d'ablation standard, un traitement personnalisé ou semi-personnalisé sur la base d'un front d'onde et un traitement sur une base topographique ou un traitement hybride sur la base du classement, l'étape d'analyse comprenant l'identification d'un paramètre de limite admissible pour chacun des algorithmes de traitement (265) disponibles et la détermination des algorithmes de traitement potentiellement exploitables en cas de dépassement des paramètres limites admissibles, et le traitement de ladite pluralité d'algorithmes de traitement potentiellement exploitables, les algorithmes de traitement disponibles recourant à un ou à plusieurs paramètres par défaut ;

la présentation (270) pour visualisation d'une pluralité de plans de traitement (290) correspondant à la pluralité d'algorithmes de traitement potentiellement exploitables ;

la modification sélective d'un ou de plusieurs paramètres par défaut et d'autres paramètres de traitement ;

le re-traitement (230') de la pluralité d'algorithmes de traitement potentiellement exploitables en recourant aux paramètres modifiés ; et

la re-présentation (270') pour une nouvelle visualisation de la pluralité de plans de traitement correspondant à la pluralité d'algorithmes de traitement potentiellement exploitables.

**24.** Procédé selon la revendication 23, où l'étape de classement comprend la détermination si l'oeil est soit a) un oeil vierge ou un oeil précédemment traité, soit b) un oeil régulier ou un oeil irrégulier, soit c) un oeil myope avec ou sans astigmatisme mixte ou un oeil hypermétrope avec ou sans astigmatisme mixte.

**25.** Procédé selon la revendication 23, où les données d'entrée sélectionnées des différents types sont mémorisées dans des fichiers différents, sélectionnables par l'utilisateur.

**26.** Procédé selon la revendication 23, comprenant en outre la sélection (292) d'un des plans de traitement.

**27.** Procédé selon la revendication 23, où les plans de traitement comprennent des données relatives à au moins un élément parmi un taille du point d'ablation laser, un positionnement du tir d'ablation laser, une séquence de tirs d'ablation laser, un fichier de tir laser, une carte simulée de front d'onde post-opératoire, une carte simulée de topographie post-opératoire, un profil simulé d'ablation, une carte kératométrique axiale, une pachymétrie cornéenne, une dimension de zone optique, une valeur de réfraction manifeste, une valeur de réfraction cible, des informations d'aberration d'ordre supérieur, une profondeur de tissu stromal résiduel et des mesures de la vision.

**28.** Procédé selon la revendication 27, comprenant en outre le tri sélectif d'au moins quelques-unes des données pour chacun des plans de traitement en fonction de critères préférentiels d'un utilisateur, comprenant au moins un élément parmi la réfraction cible, la profondeur stromale résiduelle et la dimension de zone optique.

**29.** Procédé selon la revendication 28, comprenant en outre l'optimisation d'au moins un des critères préférentiels de l'utilisateur.

**30.** Procédé selon la revendication 28, comprenant en outre la présentation sélective des données triées pour revu par l'utilisateur.

**31.** Procédé selon la revendication 23, où les algorithmes de traitement (265) disponibles comprennent au moins deux algorithmes sélectionnés dans un groupe comprenant un traitement de la myopie seulement, un traitement de l'hypermétropie seulement, un traitement de la myopie avec astigmatisme, un traitement de l'hypermétropie avec astigmatisme, un traitement correctif d'aberration d'ordre inférieur, un traitement correctif d'aberration d'ordre supérieur, un retraitement, un traitement correctif sphérique, un traitement correctif asphérique, un traitement LASIK, un traitement LASEK, un traitement PRK, un traitement avec nomogramme d'ajustement, et un traitement personnalisé.

**32.** Procédé selon la revendication 23, où le ou les paramètres par défaut représentent une valeur pour des paramètres comprenant au moins un élément parmi une zone optique, une épaisseur de volet cornéen et un autre paramètre influençant le calcul des algorithmes.

**33.** Procédé selon la revendication 23, comprenant en outre la préparation d'un dispositif d'affichage (370) d'une interface graphique d'utilisateur (GUI) destinée à être utilisée par un utilisateur.

**34.** Procédé selon la revendication 23, comprenant en outre la stockage (280) de la pluralité de plans de traitement traités à nouveau sur un support de stockage lisible par un dispositif (282).

**35.** Procédé selon la revendication 23, où l'étape de tri comprend l'optimisation d'au moins un des critères préférentiels d'utilisateur et le tri sur la base de cette optimisation.

**36.** Procédé selon la revendication 33, où l'affichage de GUI comprend en outre l'affichage sélectif d'un écran de vérification de données contenant des données récapitulatives du plan de traitement sélectionné.

**37.** Procédé selon la revendication 35, où les données récapitulatives comprennent un ou plusieurs des éléments suivants : informations d'identification de patient, plan de traitement sélectionné, réfraction manifeste, réfraction objective pour un diamètre de pupille donné, valeurs K pré-opératoires et post-opératoires ciblées, valeurs Q pré-opératoires et post-opératoires ciblées, dimension de zone optique, zone de traitement, nombre de tirs d'ablation et durée de traitement, profondeur d'ablation maximale, profondeur d'ablation centrale et pachymétrie stromale résiduelle pour une épaisseur de volet cornéen spécifié.

**38.** Procédé selon la revendication 23, où l'étape de traitement comprend en outre l'exploitation d'au moins un ensemble de données parmi les données de poursuite par rotation oculaire et les données microkératométriques.

**39.** Procédé selon la revendication 23, comprenant en outre la recommandation automatique d'une pluralité de plans de traitement préférentiels à l'utilisateur.

**40.** Procédé selon la revendication 33, où le recours à GUI comprend des options d'utilisateur sélectionnées dans un groupe comprenant la sélection de patient, le réglage de valeurs par défaut, l'affichage d'informations de logiciel de traitement, l'entrée de données de patient, et la création d'en-têtes d'écran d'affichage.

**41.** Procédé selon la revendication 33, où l'affichage de GUI comporte le codage couleur des plans de traitement, l'affichage sélectif d'informations de poursuite par rotation oculaire, l'affichage sélectif d'informations microkératométriques, la minimisation/maximisation de la taille de présentation des données, un message d'alerte basé sur une modification de paramètre sélectionné par l'utilisateur, le stockage de paramètres de plan de traitement sur un support de stockage sélectionné et autre surveillance paramétrique.

**42.** Procédé selon la revendication 23, où la pluralité de plans de traitement comprend un plan de traitement personnalisé qui réduit des aberrations de front d'onde d'ordre supérieur, et un plan de traitement non personnalisé qui améliore des aberrations d'ordre inférieur.

**43.** Procédé selon la revendication 42, où le plan de traitement non personnalisé est basé au moins en partie sur une valeur de lecture K non normalisée de l'oeil du patient.

**44.** Procédé selon la revendication 42, où le plan de traitement non personnalisé est basé au moins en partie sur un facteur Q de forme cornéenne asphérique de l'oeil du patient.

**45.** Procédé selon la revendication 42, où chacun des plans de traitement est basé au moins en partie sur une valeur d'épaisseur de stroma résiduel prospective.

**46.** Procédé selon la revendication 45, où la valeur d'épaisseur de stroma résiduel prospective est une valeur estimée.

**47.** Procédé selon la revendication 45, où la valeur d'épaisseur de stroma résiduel prospective est une valeur calculée.

FIG.1

FIG.2

$265_{a-n}$

```
┌ ─ ─ ─ ─ ─ ─ ─ ┐
│ ALGORITHM A │
│ ALGORITHM B │
│ ALGORITHM C │
│ ALGORITHM D │
│ ALGORITHM E │ 310
│      •       │
│      •       │
│      •       │
│ ALGORITHM N │
└ ─ ─ ─ ─ ─ ─ ─ ┘
```

300

(DIAGNOSTIC INPUT)

$210_a$  $210_b$  $210_n$

CALCULATION MODULE

ANALYZE DATA 220

PROCESS MULTIPLE
ALGORITHMS $260_{a-n}$

TREATMENT
PLANNING

275

240

SHOT PLACEMENT,
SHOT SEQUENCE,
SIMULATED POST-OP
MAP, OTHER
PARAMETERS

DISPLAY DEVICE 370

STORAGE DEVICE 282

SHOT FILE

399

INPUT CHANGE
PARAMETERS

$290_x$

294

LASER

(APPLICATION OF
SHOT FILE)

EYE

FIG.3

RE-CALCULATE SUCCESSFUL MULTIPLE TREATMENT
PLAN OPTIONS USING MODIFIED CACULATION
PARAMETERS;
OPTIMIZATION/SORTING ──── 250
RE-DISPLAY

286

270″

252b      252c

252a   OZ        RESIDUAL DEPTH   REFRACTION

**FIG.4**

(ALL COMBINATIONS THAT WORK)

DISPLAY/STORAGE   { 270
                    270′
                    270″

TREATMENT A         | TREATMENT B
(SHOT-PLACEMENT,
SHOT SEQUENCE,
290a   SIMULATED POST-OP                    290b
MAP, OTHER
PARAMETERS; SHOT FILE)

**FIG.5**

290c   TREATMENT C      TREATMENT D   290d

**FIG.6**

TABLE

| PARAMETER | POSSIBLE RANGE | TYPE |
|---|---|---|
| SPHERE | −25 D TO +10 D | INPUT |
| CYLINDER | −10 D TO +10 D | INPUT |
| AXIS | 0° TO 180° | INPUT |
| K−READING | 33 D TO 53 D | INPUT |
| OPTICAL ZONE | 4.5mm TO 8.5mm | INPUT |
| FLAP THICKNESS | $0\mu m$ TO $300\mu m$ | INPUT |
| PACHYMETRY | $300\mu m$ TO $700\mu m$ | INPUT |
| PUPILLOMETRY | 4mm TO 11mm | INPUT |
| MAX. ABLATION | UP TO $250\mu m$ | OUTPUT |
| NUMBER OF PULSES | UP TO 9000 | OUTPUT |

**FIG.19**

Smith, John
OD: Date: ____

Treatment | Treatment

Information
Rotational Eye Tracker Information
Recommendation Information
Restore Defaults

ess (μm): 180 ▽    Optical Zone (mm): 6.5 ▽    Pachymetry (μm): 523

Ⓑ Zyoptix B
Ⓒ Zyoptix C
Ⓓ Zyoptix D
▦ Calculate
🖫 Zyoptix Export
🖨 Print

◁ ‖ ?

**Zyoptix A**

0.12 μm
523 μm
Accoustic Factor: 0.92+(Pac + 0)

Subjective Refraction: −2/0.5/10°
Zywave PPR: 2.47/0.63/11°
Zywave Pupil Diameter: 6mm

Treatment
Sphere: −2.47 dpt
Cylinder: 0.63 dpt
Axis: 11°
Number Of Pulses: 2144
Treatment Time: ????
Calculated Central Ablation: 45 μm

Ablation Profile ▽
10mm
6mm
2mm

Max Ablation: 50 μm
Remaining Stroma: 303 μm
Optical Zone: 6.50 mm

**Zyoptix B**

NORMS (6mm): 0.32 μm
Pachymetry: 523 μm
Accoustic Factor: 0.92+(Pac + 0)

Subjective Refraction: −2/0.5/10°
Zywave PPR: 2.47/0.63/11°
Zywave Pupil Diameter: 6mm

Treatment
Sphere: −2.47 dpt
Cylinder: 0.63 dpt
Axis: 11°
Number Of Pulses: 1744
Treatment Time: ????
Calculated Central Ablation: 45 μm

Ablation Profile ▽
10mm
6mm
2mm

Max Ablation: 47 μm
Remaining Stroma: 313 μm
Optical Zone: 6.50 mm

**Zyoptix C**

PreOp K−Reading: 44.1 dpt
PreOp Conic Const: −0.17
Pachymetry: 523 μm
Accoustic Factor: 0.92−(Pac + 0)

Subjective Refraction: −2.0/0.5/10°

Treatment
Sphere: −2.0 dpt
Cylinder: 0.5 dpt
Axis: 10°
PreOp K−Read: 44.1 dpt
PreOp Conic: −0.17
Desired: −0.56
Number Of Pulses: 2052
Treatment Time: ????

Ablation Profile ▽
10mm
6mm
2mm

Max Ablation: 50 μm
Remaining Stroma: 910 μm
Optical Zone: 6.50 mm

**Zyoptix D**

PreOp K−Reading: 44.1 dpt
PreOp Conic Const: −0.17
Pachymetry: 523 μm
Accoustic Factor: 0.92−(Pac + 0)

Subjective Refraction: −2.0/0.5/10°

Treatment
Sphere: −2.0 dpt
Cylinder: 0.5 dpt
Axis: 10°
PreOp K−Read: 44.3 dpt
PreOp Conic: −0.17
Desired: −0.56
Number Of Pulses: 1922
Treatment Time: ????

Ablation Profile ▽
10mm
6mm
2mm

Max Ablation: 46 μm
Remaining Stroma: 314 μm
Optical Zone: 6.50 mm

313

## FIG.7

EP 1 613 253 B1

314

## FIG.8

BAUSCH & LOMB          OS 03/29/2001    Doe, John

Data Check

Save

Cancel

Birthdate: 12/28/1988                    Treatment Planner
                                         Version 0.02

┌─Pachymetry ──────────────────┐  ┌─Zywave Exam Information ──────────┐
│ User Selected Pachymetry:     │  │ Zywave Exam Date: 12/28/2002 12:33:45 PM │
│ Orbscan Pachymetry: 523 μm    │  │ Zywave PPR (6mm): 2.47/ 0.63/11'  │
│ Orbscan Exam Date: 12/28/2002   12:33:45 PM │  │ Zywave RMS (6mm): ????  │
│ Remaining Pachymetry Under Flap: ???? │  │ Rotational Eye Tracker Used │
│ Flap Thickness: 160 μm        │  │                                   │
└───────────────────────────────┘  └───────────────────────────────────┘

┌─Zyoptix A Treatment ────────────────────────03/21/2003 2:32:22 PM─┐
│ Sphere: −2.47 dpt      Manifest Refraction: ????    Optical Zone: 6.5 mm  │
│ Cylinder: −0.63 dpt    Number Of Pulses: 2144       Treatment Zone: ????  │
│ Axis: 11'              Laser Frequency: 50 Hz       Max Ablation: 50μm    │
│                        Treatment Time: ????         Central Ablation: 45μm │
└──────────────────────────────────────────────────────────────────┘

315a

◁ ▯ ?                                          Demo Version

**FIG.9A**

BAUSCH & LOMB          OD 01/23/2002    Doe, Jane

Data Check

Save

315b

Cancel

Birthdate: 12/28/1988                    Treatment Planner
                                         Version 0.02

┌─Pachymetry ──────────────────┐
│ User Selected Pachymetry:     │
│ Orbscan Pachymetry: 523 μm    │
│ Orbscan Exam Date: 12/28/2002 │
│ Remaining Pachymetry Under Flap: ???? │
│ Flap Thickness: 160 μm        │
└───────────────────────────────┘

┌─Zyoptix C Treatment ────────────────────────03/21/2003 2:32:22 PM─┐
│ Sphere: −2.47 dpt      Manifest Refraction: ????    Optical Zone: 6.5 mm  │
│ Cylinder: −0.63 dpt    Number Of Pulses: 2144       Treatment Zone: ????  │
│ Axis: 11'              Laser Frequency: 50 Hz       Max Ablation: 50μm    │
│ PreOp K−Read: 44.1 dpt  Treatment Time: ????        │
│ Q Pre: −0.17           │
│ Q Post: ????           │
└──────────────────────────────────────────────────────────────────┘

◁ ▯ ?                                          Demo Version

**FIG.9B**

FIG.10

COLOR CODED 317

| Treatment | Doe, Jane<br>OD: Date: ____ | "Zyoptix A Treatment" |

Treatment Review

Calculate

Zyoptix Export

Print

Flap Thickness (μm): [180 ▽]

NORMS (6mm): 0.32 μm
Pachymetry (μm): [523]
Accoustic Factor: 0.92' (PAC + 0)

Subjective Refraction: −2/0.5/10'
Zywave PPR: −2.47/0.63/11'
Zywave Pupil Diameter: 6 mm

**Ablation Profile** ▽

10mm
6mm
2mm

**Treatment**

| Sphere (dpt): | [−2.47] [100 %] | Max Ablation: | 50 μm |
| Cylinder (dpt): | [−0.63] [100 %] | Remaining Stroma: | 303 μm |
| Axis ('): | 11' | Optical Zone: (mm): | [6.5 ▽] |
| Number of Pulses: | 2144 | Calculated Central Ablation: | 45 μm |
| Treatment Type: | ???? | | |

◁ ▮ ?

## FIG.11A

NOT COLOR CODED 317

| Treatment | Doe, Jane<br>OD: Date: ____ | Zyoptix B Treatment |

Treatment Review

Calculate

Zyoptix Export

Print

Flap Thickness (μm): [180 ▽]

NORMS (6mm): 0.32 μm
Pachymetry (μm): [523]
Accoustic Factor: 0.92' (PAC + 0)

Subjective Refraction: −2/0.5/10'
Zywave PPR: −2.47/0.63/11'
Zywave Pupil Diameter: 6 mm

**Ablation Profile** ▽

10mm
6mm
2mm

**Treatment**

| Sphere (dpt): | [−2.47] [100 %] | Max Ablation: | 47 μm |
| Cylinder (dpt): | [−0.63] [100 %] | Remaining Stroma: | 313 μm |
| Axis ('): | 11' | Optical Zone: (mm): | [6.5 ▽] |
| Number of Pulses: | 1744 | Calculated Central Ablation: | 45 μm |
| Treatment Type: | ???? | | |

◁ ▮ ?

## FIG.11B

FIG.12A

FIG.12B

BAUSCH & LOMB

1304

Select Patient Files

1318 1310

New Patient

Locate Files

Open

1302

Patient Search

Last Name [                    ]   First Name [                    ]

OTE Files

| Smith, John | OD | 03/18/2003 11:43:22 AM | . |
|---|---|---|---|

1304

1306

1308

1312

DOB (mm/dd/yyyy):
Gender:
Sub Refraction:
File Name:
Comment:

1316

ATE Files

| Smith, John | OD | 09/17/2003 16:34:25 |
|---|---|---|

Patient ID:
DOB (mm/dd/yyyy):
Gender:
Sub. Ref.:
PPR:
Wavefront Dia.(mm):
File Name:
Comment:

◁ 〗 ?

Zyoptix Treatment Planner

## FIG.13

BAUSCH & LOMB

Patient Information

Continue

Restore

Cancel

1402

Patient Information

Patient ID
[000011]

Last Name
[Smith]

First Name
[John]

DOB (mm/dd/yyyy)        Age
[05/27/1967]             36

Gender
⊙ Male    ○ Female

Eye Information

Eye
⊙ OD   ○ OS        ☐ Retreatment

Subj. Sphere        PPR Sphere
[-2.50]             [-2.39]

Subj. Cylinder      PPR Cylinder
[-1.25]             [-1.32]

Subj. Axis          PPR Axis
[170]               [163]

Pachymetry (µm)     Pupillometry (mm)
[616]               [6.4]

◁ 〗 ?

Zyoptix Treatment Planner

## FIG.14

**FIG.15**

**FIG.16**

BAUSCH & LOMB | Smith, John OD

Options | Zyoptix Personalized Treatment

Treatment Overview
Maps
Calculate
1615— Zyoptix Export

1702—

HO-RMB (6mm): 0.49 μm
Wavefront Diameter: 7.01 mm
Pupillometry: 6.4 mm
Pachymetry: 616 μm
Subj. Refraction: −2.50/−1.25/170

Ablation Profile

Treatment
PPR: −2.39 / −1.32 / 163
Treatment Refraction: −2.39 −1.32 163
Percent PPR: 90 100

Optical Zone (mm): 6.9
Flap Thickness (μm): 160
Max. Ablation: 61 μm
Central Ablation: 61 μm
Residual Stroma: 388 μm Est.
Treatment Zone: 9.2 x 9 mm
Pulses (time): 2495 (25 sec)
Messages:

○ Calc.

56.7
50.2
43.6
37.1
30.6
24
17.5
10.9
4.4

◁ ▯ ?

Zyoptix Treatment Planner

**FIG.17**

BAUSCH & LOMB | Smith, John OD

Data Check

Save
Print
Save and Print
Treatment Overview
1802a— Select Patient
Finished

Surgeon Name: Dr. Theisen

Patient-ID: 000011
Last Name: Smith
First Name: John
Gender: Male
Birthdate: 5/27/1967
Age: 36
Eye: OD

Treatment Planner Ver. 1.7 (1430.30837)
Location:
DLL Ver: 1.2
Wave Exam File: JohnSmithOD-TP.ATE
Wave Exam Date: 9/17/2003 4:34 PM
Wave Serial Number: 1206
Topo Exam File: JohnSmithOD-TP.ots
Topo Exam Date: 3/18/2003 11:43 AM
Topo Serial Number: 99284693

Planned Treatment − Zyoptix Tissue Saving ──────── Dec 18,2003 1:31 PM ──────

Procedure: Tissue Savings
Laser Frequency: 100 Hz
Treatment Type: Myopia
Sub. Ref. Type: Myopia
Subjective Refraction: −2.50/−1.25/170
Treatment Refraction: −2.50/−1.25/170
Percent of Sub. Ref.: 100%

K-Value Input: 43.3 D
Optical Zone: 6 mm
Max Ablation: 52 μm

Topo Accoustic Factor: ----
Topo Central Pachy: 616 μm
Topo Thinnest Pachy: ----
Entered Pachymetry: ----
Flap Thickness: 160 μm
Est. Residual Stroma: 397 μm
Estimated from Post-Op Pachymetry Map and Flap Thickness.

◁ ▯ ?

Zyoptix Treatment Planner

**FIG.18A**

BAUSCH & LOMB  Smith, John OD

Data Check

1804

Save
Print
Save and Print
Treatment Overview
Select Patient
Finished

1802b

| Laser Frequency: | 100 Hz | Topo Thinnest Pachy: | ---- |
| Treatment Type: | Myopia | Entered Pachymetry: | ---- |
| PPR Type: | Myopia | Flap Thickness: | 160 μm |
| Subjective Refraction: | -2.50/-1.25/170 | Est. Residual Stroma: | 395 μm |
| PPR (3.5mm): | -2.39/-1.32/163 | | |
| Treatment Refraction: | -2.39/-1.32/163 | Estimated from Post-Op Pachymetry Map and Flap Thickness. | |
| Percent of PPR: | 100%/100% | | |
| | | | |
| Optical Zone: | 6 mm | | |
| Max. Ablation: | 54 μm | | |
| Central Ablation: | 54 μm | Iris Recognition: | Available |
| Treatment Zone: | 8.3 x 8.2 mm | Avg Pupil shift X/Y: | 26 μm / 80 μm |
| Number Pulses (Time): | 1856 (19sec) | Avg Angle dil. vs undil.: | -0.35° |
| Pulses for 2mm/1mm: | 1317/539 | | |
| Num. Treatment Phases: | 2 | TLB file: | |

Messages

Recommended Centration is the Pupil Center.

| Entered Pupillometry: | ---- |
| Wave Pupillometry: | 6.4 mm |
| | |
| Wavefront Diameter: | 7.01 mm |
| HO-RMS (6mm): | 0.49 μm |
| Z400 (6mm): | -0.36 μm |

◁ ⫯ ?

Zyoptix Treatment Planner

**FIG.18B**

BAUSCH & LOMB  Smith, John OD

Data Check

Save
Print
Save and Print
Treatment Overview
Select Patient
Finished

1802c

| Pulses for 2mm/1mm: | 1317/539 | | |
| Num. Treatment Phases: | 2 | TLB file: | |

Messages

Recommended Centration is the Pupil Center

| Entered Pupillometry: | ---- |
| Wave Pupillometry: | 6.4 mm |
| | |
| Wavefront Diameter: | 7.01 mm |
| HO-RMS (6mm): | 0.49 μm |
| Z400 (6mm): | -0.36 μm |

Print Comments

| Pre-Op Q (Conic,6mm): | -0.21 |
| Pre_op K-Reading(6mm): | 45.5 D |
| White to White: | ---- |
| Simks: | 46 D & 44.6 D |
| Topo Astig.: | -1.4 D @ 168° |
| Angle Kappa: | ---- |
| Pup. Center vd Apex: | ---- |

Visual Acuity SC:
Visual Acuity CC:
Planned Ring Size:

◁ ⫯ ?

Zyoptix Treatment Planner

**FIG.18C**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020075451 A1
- US 5891132 A

- US 46080103 A
- US 10100782 B